(19) **Europäisches Patentamt / European Patent Office / Office européen des brevets**

(11) **EP 4 459 287 A1**

(12) # EUROPEAN PATENT APPLICATION

(43) Date of publication:
**06.11.2024   Bulletin 2024/45**

(21) Application number: **23171184.7**

(22) Date of filing: **02.05.2023**

(51) International Patent Classification (IPC):
**G01N 33/58** (2006.01)    **G01N 33/68** (2006.01)

(52) Cooperative Patent Classification (CPC):
**G01N 33/6845; G01N 33/582**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Synsight**
  **"Génopole Entreprises"**
  **91058 Évry-Courcouronnes (FR)**
- **INSTITUT NATIONAL DE LA SANTE ET DE LA RECHERCHE MEDICALE - INSERM**
  **75013 Paris (FR)**
- **Université d'Evry Val d' Essonne**
  **91000 Evry-Courcouronnes (FR)**

(72) Inventors:
- **BOLLOT, Guillaume**
  **44300 NANTES (FR)**
- **BAUVAIS, Cyril**
  **77190 DAMMARIE-LES-LYS (FR)**

- **PASTRE, David**
  **91450 SOISY-SUR-SEINE (FR)**
- **MOLLIEX, Amandine**
  **91330 YERRES (FR)**
- **DUEZ, Julien**
  **91540 MENNECY (FR)**
- **SANCHEZ, Maxime**
  **75019 PARIS (FR)**
- **BABAULT, Nicolas**
  **75015 PARIS (FR)**
- **CRAVEUR, Pierrick**
  **91270 VIGNEUX-SUR-SEINE (FR)**
- **HENRIE, Hélène**
  **78120 RAMBOUILLET (FR)**

(74) Representative: **Yes My Patent**
**32 Boulevard Richard Lenoir**
**75011 Paris (FR)**

Remarks:
The complete document including Reference Table(s) and the Sequence Listing(s) can be downloaded from the EPO website

(54) ## METHODS AND TOOLS FOR DETECTING THE IMPACT OF COMPOUNDS ON PROTEIN, RNA OR DNA INTERACTIONS

(57)    The present invention covers an in vitro method for evaluating the ability of a compound to disrupt or stabilize an interaction between one or more bait and one or more candidate prey in a eukaryotic cell. In addition, the present invention covers compounds identified by the *in vitro* method according to any one of the preceding claims, for use as a medicament.

[Fig. 1]

**EP 4 459 287 A1**

## Description

The present invention covers an in vitro method for evaluating the ability of a compound to disrupt or stabilize an interaction between one or more bait and one or more candidate prey in a eukaryotic cell. In addition, the present invention covers compounds identified by the *in vitro* method according to any one of the preceding claims, for use as a medicament.

## Technical Field

The invention relates to methods, uses and tools for studying ligand interactions in a eukaryotic cell, and the impact of compounds on said interactions, preferably in order to identify new medicament candidates.

## Background Art

Protein-protein interactions (PPIs) are crucial for cellular functions and present in all type of organisms with an estimated of >500,000 interactions in human (Li et al., 2017; Rolland et al., 2014). PPIs are involved in the regulation of biological pathways which is consistent with their implication in the development of numerous disorders. Therefore, they represent appealing targets for the development of new therapeutics to treat various human diseases (Lu et al., 2020; Scott et al., 2016). Most PPI interactions have firstly been coined undruggable because of their large surface of interaction before appearing as potential targets full of promises with the identification of several efficient inhibitors of PPI related to human diseases (Arkin and Wells, 2004; Arkin et al., 2014).

Targeting RNA:Protein Interactions (RPIs) critically involved in pathological mechanisms is also a promising strategy to find novel classes of drug candidates that remains largely unexploited (Einstein, J. M. et al., 2021). RPIs in cells are highly diverse encompassing interactions with messenger RNA (mRNA) (Baltz, A. G. et al. 2012), ribosomal RNA (rRNA) (Simsek, D. et al. 2017), and non-coding RNA (ncRNA)(Lu, S. et al. 2019), which are critical to fine tune the spatiotemporal gene expression. As revealed by genomic approaches (Van Nostrand, E.L. et al. 2020 & Castello, A. et al. 2012), the human genome contains more than 1000 transcripts encoding RNA-Binding Proteins (RBPs), thus providing a large variety of interactions with coding or non-coding RNAs. However, while the diversity of RNA:Protein interfaces may allow the development of RPIs inhibitory molecules (Wu, P. 2020), only scarce studies have already been undertaken and were restricted to few complexes such as Lin28-let7 interactions (Roos, M. et al. 2018 & Wang, L. et al. 2018), MSI-RNA (Minuesa, G. et al. 2019) and hnRNP A18- RNA (Solano-Gonzalez, E. et al. 2021).

As mentioned, targeting PPIs and RPIs for drug discovery are attractive strategies for the pharmaceutical industry. However, despite many years of extensive research to develop new PPI or RPI modulators, only a couple small molecules have made their way into the market. While the large surface area of PPI and RPI can make drug development difficult, the availability of methods to accurately assess with high-content screening approaches whether candidate modulators are effective in a cellular setting is lacking.

In particular regarding PPIs, because of the abovementioned obstacles along the way of PPI inhibitor discovery, the needs of methods to perform large-scale screen of putative PPI inhibitors is particularly acute. To this end, several *in vitro* and cell-based methods have already been developed (Arkin et al.; Titeca et al., 2019; Wade and Arkin). One of the most popular assays for screening small molecules targeting specific protein-protein interactions is the Homogeneous Time-Resolve Fluorescence (HTRF) (Degorce, 2009). This extremely sensitive technology based on energy transfer between a donor and an acceptor molecule has been adapted to automation for HTS. However, the major drawbacks of HTRF, like for others *in vitro* methods, is the necessity of purifying the proteins of interest, which most of the time is limited to the use of specific domains, and their lack of information regarding the fate of small molecules and their efficiency to inhibit PPIs in a cellular environment. While HTRF method is still able to monitor protein-protein interactions in cells, the adaptation of HTRF to cell-based assays required a latter step of protein labeling with antibodies or chemical linkage that decrease the interest of using this approach in cell assays (Degorce, 2009). Although FRET techniques were primarily used to assess the interaction between two proteins in cells, the lack of sensitivity and a low signal to noise ratio rendered it unsuitable for accurately measuring the disruption of interactions in a cellular environment (Piston and Kremers, 2007). In the other hand, BRET which is still based on energy transfer but uses bioluminescence instead of fluorescence, has been the subject of renewed interest with the recent development of the nanoluciferase. This small, bright, and stable donor has vastly broadened the potential applications of BRET assays with its use in NanoBRET assay (Dale et al., 2019; Machleidt et al., 2015). NanoBRET Technology is based on the expression of full-length proteins tagged with the bright luciferase Nanoluc which serve as the energy donor and a fluorophore called HaloTag as the acceptor, the readout is the average luminescent signal per well recorded on a microplate reader. This technology enables detection of PPI in the natural cellular environment only when the donor and acceptor probes are sufficiently close to each other to permit the energy transfer. In addition, even though NanoBRET assay has a good sensitivity, its reproducibility relies upon the expression of a donor since BRET signal is dependent of the donor/acceptor ratio as described by the donor saturation assay (Couturier and Deprez, 2012). To overcome this limitation and ensure the stability and reproducibility of the BRET signal, stable cell lines expressing the donor are preferred to develop a BRET-based screening assay, increasing the complexity of setting up such assays (Couturier and Deprez, 2012). Thus, there is a need for a reliable and simple cell-based assay that could be used to screen small molecules modulators of protein-protein interactions.

Regarding RPis, to find potent inhibitors of RNA:protein interfaces, previous approaches used *in vitro* assays such as fluorescence polarization assay complemented by pull-down experiments with cell lysates or RNA ELISA to test the effectiveness or selectivity of few hits (Roos, M. et al., 2016 & Minuesa, G. et al.). While *in vitro* approaches are important to define putative hits and lead to the validation of effective compounds, deciphering whether the selected molecules are effective in a cellular context generally relies on indirect measurements using techniques such as Cellular Engagement Thermal Shift Assay (CETSA) or functional assays where the putative consequences of disrupting RPIs on cellular function bear a considerable uncertainty. Indeed, multiple functions are associated to RBPs, which renders the interpretation of the results of functional assays tricky. In addition, toxicity and off-target effects are putative biases which are always difficult to get rid of, notably when using small molecules with $K_D$ in the low micromolar range, which is generally the case for RPI inhibitors (Jung, J. et al. 2013 & Camborde, L. et al. 2017). To fill the gap between *in vitro* and functional assays, cellular approaches initially used to detect PPIs such as FRET (Fluorescence Resonance Energy Transfer) or PLA (Proximity Ligation Assay) have been adapted to detect RPIs (Jung, J. et al. 2013 & Camborde, L. et al. 2017) in cells but several technical issues have hampered their application such as the requirement of an adapter to RNA in FRET and PLA, the proximity of the donor and acceptor proteins in FRET, and the use of antibodies in PLA.

Besides assays to probe RPIs in cells, other challenges arise from the drug discovery process such as the quality of the computational models, the strategies used in the *in silico* screening, and the lack of experimental feedback and validation of computationally predicted inhibitors essential to orient the rational drug design procedure toward the most relevant molecules.

The WO2016012451 patent family, filed partly by the same inventors of the present invention, relates to methods for detecting an interaction between one or more protein bait and one or more candidate prey in a eukaryotic cell, by the use of a polymerized-tubulin binding moiety, such as a Microtubule-Binding Domain (MBD), fused to one or more bait, as a tool for determining the occurrence of an interaction between said one or more bait and one or more candidate prey in an eukaryotic cell.

Starting from the technology described in this patent family, the applicants have developed an innovative new technology, which allow to assess the ability of a compound to disrupt or stabilize interactions between DNA, RNA and/or proteins, and in particular to assess the ability of a compound to disrupt or stabilize PPIs and RPIs by single cell fluorescence microscopy on a High-Content Screening (HCS) imager. This technique allows to screen molecule libraries, in order to identify medicament candidates.

This technique is able to tackle the above challenges by introducing an experimental assay amenable to HCS to score interactions, in particular RPIs or PPIs, in cells and a drug screening approach that integrates chemical, structural and cellular data from experimental techniques for the identification and development of molecules, in particular small molecules, that target interactions, in particular RPIs or PPIs.

## Summary of Invention

The present invention relates to methods for evaluating the ability of a compound to disrupt or stabilize an interaction between one or more bait, preferably protein, and one or more candidate prey in a eukaryotic cell, and more particularly in a living eukaryotic cell context.

The system consists of a detection system based on the expression in cells of (i) a "bait", a known protein, ribonucleic acid or deoxyribonucleic acid, which is brought to microtubules thanks to its fusion to a microtubule-binding domain and (ii) a prey which can be for instance a protein, a nucleic acid or a deoxyribonucleic acid.

The prey, when interacting with the bait, becomes also localized along microtubules, which makes it thus easily detectable. As developed in the present invention, it allows to determine a difference in interaction before and after contact with a test molecule and to identify molecules of interest in various pathologies, for example where PPIs and RPIs are implicated, such as cancers, viral or bacterial infectious diseases, epigenetic diseases or proteinopathies.

Here, the system of WO2016012451 (MT BENCH) was improved to fill the gap between *in vitro* and functional assays by probing whether the interaction of a selected bait and a selected prey is affected in a cellular context by a compound.

The invention further relates to baits, vectors, cell lines, kits suitable for said methods, uses, and compound identified according to the method of the invention.

Thus, a first aspect of the invention is to provide an *in vitro* method for evaluating the ability of a compound to disrupt or stabilize an interaction between one or more bait and one or more candidate prey in a eukaryotic cell, comprising the steps of:

a. providing a eukaryotic cell expressing (i) one or more bait, and (ii) one or more candidate prey, wherein said bait comprises a bait moiety and a polymerized tubulin-binding moiety;

b. determining the occurrence of an interaction between said one or more bait and said one or more candidate prey in the eukaryotic cell, wherein said bait is bound to polymerized tubulin in the eukaryotic cell, thereby localizing said one or more candidate prey along said polymerized tubulin, thereby detecting said interaction;

c. contacting the said eukaryotic cell with the said compound;

d. determining the occurrence of the interaction between said one or more bait and said one or more candidate prey in the eukaryotic cell as in step b. and quantify this interaction;

e. Comparison of the occurrences of the interaction made in step b. and in. step d.;

f. Conclude whether the effect of the said compound is the disruption or the stabilization of the interaction between said one or more bait and one or more candidate prey.

In a preferred embodiment, said one or more bait is a protein.

In a preferred embodiment, one or more candidate prey is a protein or a ribonucleic acid.

A second aspect of the invention relates to the use of a polymerized-tubulin binding moiety fused to one or more bait, as a tool for evaluating the ability of a compound to disrupt or stabilize an interaction between said one or more bait and one or more prey in a eukaryotic cell, wherein said bait is bound to polymerized tubulin in the eukaryotic cell, thereby localizing said one or more candidate prey along said polymerized tubulin, the said one or more bait and the said one or more candidate prey being contacted with the said compound, thereby allowing the evaluation of the disruption or stabilization of said interaction

A third aspect of the invention relates to a protein bait comprising a polymerized-tubulin binding moiety comprising one or more Microtubule-Binding Domain(s), and a bait moiety.

A fourth aspect of the invention relates to a ribonucleic acid bait comprising polymerized-tubulin binding moiety comprising one or more Microtubule-Binding Domain(s), and a bait moiety.

A fifth aspect of the invention relates to a deoxyribonucleic acid bait comprising polymerized-tubulin binding moiety comprising one or more Microtubule-Binding Domain(s), and a bait moiety.

A sixth aspect of the invention relates to a vector containing an expression cassette suitable for expressing a protein bait of the invention.

A seventh aspect of the invention relates to a vector containing an expression cassette suitable for expressing a ribonucleic acid bait of the invention.

An eighth aspect of the invention relates to a vector containing an expression cassette suitable for expressing a deoxyribonucleic acid bait of the invention.

A nineth aspect of the invention relates to a vector containing an expression cassette suitable for expressing a protein prey of the invention.

A tenth aspect of the invention relates to a vector containing an expression cassette suitable for expressing a ribonucleic acid prey of the invention.

An eleventh aspect of the invention relates to a vector containing an expression cassette suitable for expressing a deoxyribonucleic acid prey of the invention.

A twelfth aspect of the invention relates to cell lines that are transfected with a vector of the invention, and/or that stably express a bait suitable for the methods and uses of the invention.

A thirteenth aspect of the invention relates to cell lines that are transfected with a vector of the invention, and/or that stably express a prey suitable for the methods and uses of the invention.

A fourteenth aspect of the invention relates to kits for evaluating the ability of a compound to disrupt or stabilize an interaction between said one or more bait and one or more prey in a cell, comprising a bait, a vector, and/or a cell line suitable for the methods and uses of the invention.

A fifteen aspect of the invention relates to a compound identified by the in vitro method according to the invention, for use as a medicament.


**Brief Description of Drawings**

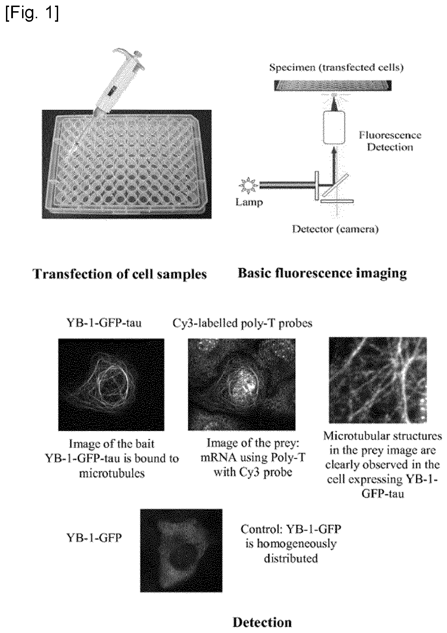

[Fig. 1] Principle of the method's steps a. and b.: high throughput analysis and detection system. Application to an endogenous mRNA interaction with YB-1, a mRNA-binding protein. (A) cells cultivated on microplates are transfected by an automate system. (B) after image acquisition, the identification of a "microtubule-like pattern" in the fluorescence channel corresponding to the prey (messenger RNA) certifies the interaction between the bait (YB-1-GFP-tau) and the prey (mRNA). (C) (upper panel; from left to right) Detection of the bait: YB-1-GFP-Tau; Detection of the prey: mRNA ; Example of the detection of a protein-RNA interaction showing the presence of a microtubule-like pattern in the Cy3 channel (corresponding to the Poly-T RNA Cy3-labeled probe to detect mRNA). (lower panel) YB-1-GFP is used as a control and is homogenously distributed in the cytoplasm.

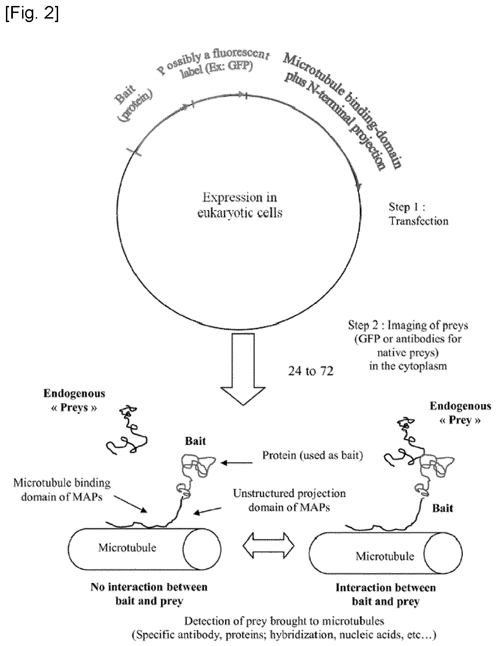

[Fig. 2] Principle of the method for fixed cells and for an endogenous prey, for steps a. and b. Detection of an endogenous prey brought to microtubules, in two steps. A first step (Step 1) includes the transfection of an expression vector coding for a protein bait comprising a Microtubule binding domain and optionally a fluorescent label. A second step (Step 2) includes the detection of the endogenous prey using reagents such as antibodies directed against the native prey or hybridization with nucleic acids. The prey is then detected on the microtubules after its interaction with the bait.

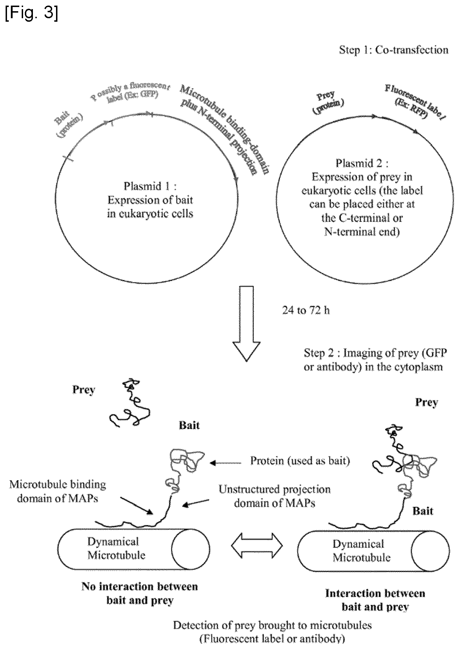

[Fig. 3] Principle of the method for living cells and for an exogenous prey for steps a. and b. A first step (Step 1) includes the co-transfection of two expression vectors; a first plasmid expresses a protein bait comprising a Microtubule binding domain and optionally a fluorescent label. A second plasmid expresses the exogenous prey optionally labelled with a fluorescent label. A second step (Step 2) includes the detection of the exogenous prey along dynamic microtubules or in the cytoplasm using a fluorescent label specific for the prey and/or an antibody.

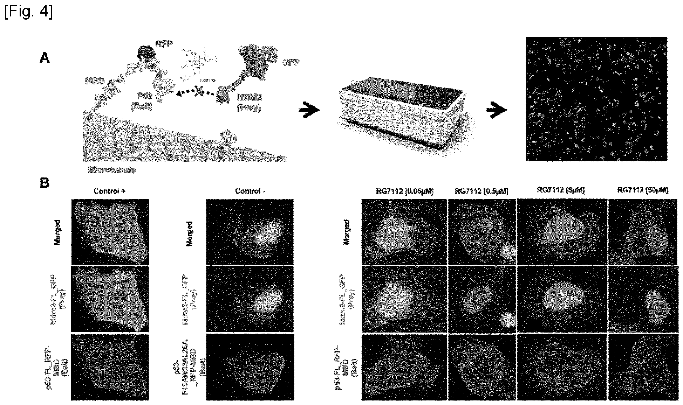

[Fig. 4] Development of High-Content Screening conditions for a case study of PPI (p53/MDM2) in a cellular context, by using the microtubule bench (MT bench). (A) Schematic representation of the MT bench technology to detect PPI modulation with small molecules. The bait (P53) is covalently bounded to RFP (Red Fluorescent Protein) and a MBD (Microtubule Binding Domain). Thus, the RFP signal follows microtubule network. The prey, (MDM2) is bounded to GFP (Green Fluorescent Protein), but free in the cytoplasm. Both signals showing the same microtubule network means that the bait and prey are interacting and can be modulated by an inhibitor (such as RG7112). This interaction can be monitored by HCS (Opera Phenix from Perkin Elmer) resulting in a high number of single cell events per well (here the representation of 10% of a 96-well surface). (B) Cellular images of fixed U-2 OS cells overexpressing p53-FL_C_RFP-MBD (used as bait protein) for the positive control (Control +), or the triple mutant p53-F19AW23AL26A_RFP-MBD (used as bait protein) for the negative control (Control -), and MDM2-FL_C_GFP (used as prey protein). Control + images show bait/prey interaction (both fluorescent signals show the same microtubule network), while Control - images show no interaction (both fluorescent signals do not show same cellular compartment), in this case MDM2 is located into nucleus. Inhibition of the interaction between p53 and MDM2 were induced by treatment with increasing concentrations of RG7112, a well-known MDM2-inhibitor (Vu et al., 2013) ([0.05μM], [0.5μM], [5μM] and [50μM]), showing the disruption of p53/MDM2 interaction and the re-localization of MDM2 in the nucleus in a dose-dependent manner.

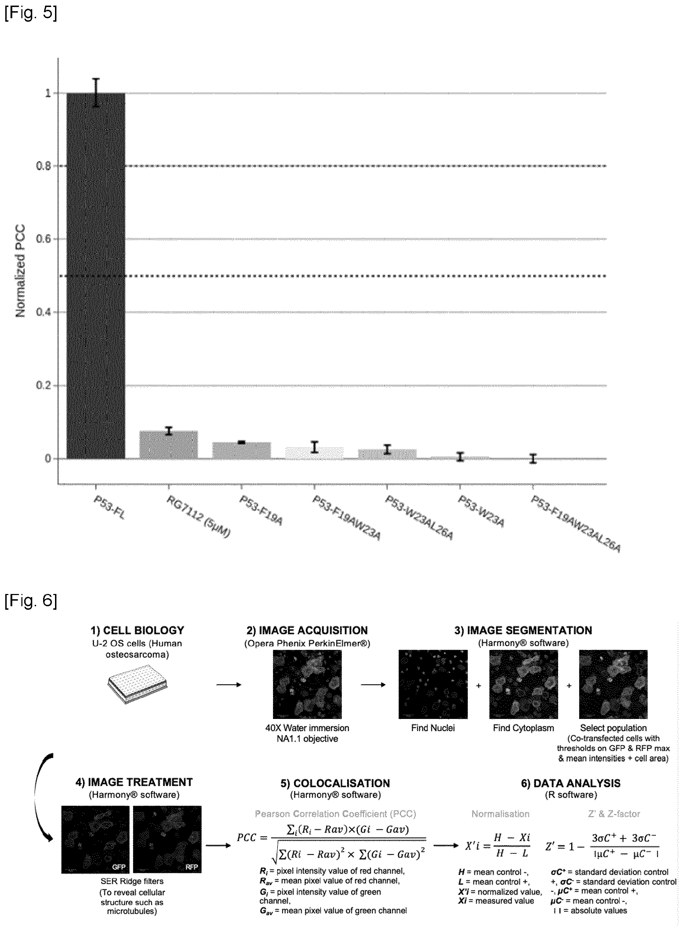

[Fig. 5] Effect of point mutations on p53 residues in its MDM2-binding site, by using the MT bench technology. Comparison of the PCC score between the wild type and different mutant constructs of p53 as bait with single (p53-F19A_C_RFP-MBD, p53-W23A_C_RFP-MBD), double (p53-F19AW23A_C_RFP-MBD, p53-W23AL26A_C_RFP-MBD) and triple mutations (p53-F19AW23AL26A_C_RFP-MBD) were normalized to allow a comparison between experimental plates (Kevorkov and Makarenkov, 2005). The PCC values were normalized relatively to the controls, by using the mean of negative control wells and the mean of positive control wells corresponding to the p53-FL/MDM2 interaction and the negative control wells with triple mutant.

[Fig. 6] Workflow of the analysis pipeline. The analyze used in this study was developed by using Harmony® and R softwares. The different stages of the pipeline are: 1) Cell biology, 2) Image acquisition, 3) Image segmentation, 4) Image treatment, 5) Co-localization and 6) Data analysis.

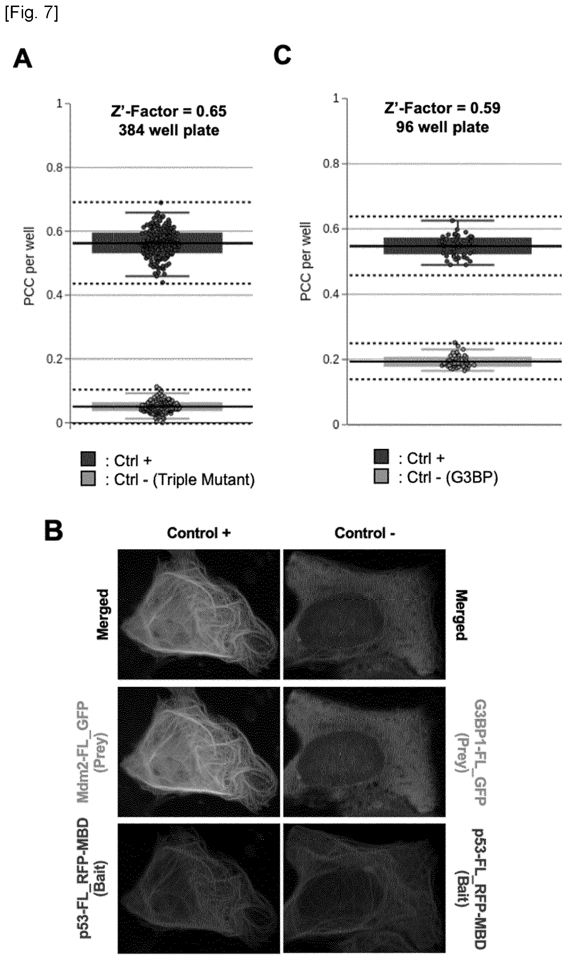

[Fig. 7] Development of the screening conditions. (A) Z'-factor graphical representation, showing the robustness of the system developed here on 384 well plate format. Z'-factor with p53 and MDM2 proteins, used as positive control (p53-FL_C_RFP- MBD/MDM2-FL_C_GFP). (B) Fixed U-2 OS cells overexpressing p53-FL_C_RFP-MBD (used as bait protein, in red) and MDM2-FL_C_GFP (used as prey protein) for the positive control (Control +), or G3BP1_GFP (used as prey protein) for the negative control (Control -). In the positive control, p53/MDM2 interact, showing in the merged channels (both fluorescent signals show the same microtubule network). On the contrary, in the negative control, p53/G3BP1 do not interact. G3BP1 protein is diffuse in the cytoplasm and not relocated on the microtubule network (both fluorescent signals do not show same microtubule network). Cells were acquired on an HCS microscopy system (Opera Phenix from PerkinElmer) with the 40X water immersion (1.1 NA) objective and confocal mode. (C) Z'-factor graphical representation, using a non-P53 related protein G3BP1 as a negative control. Graphs and computations were done using R sofware.

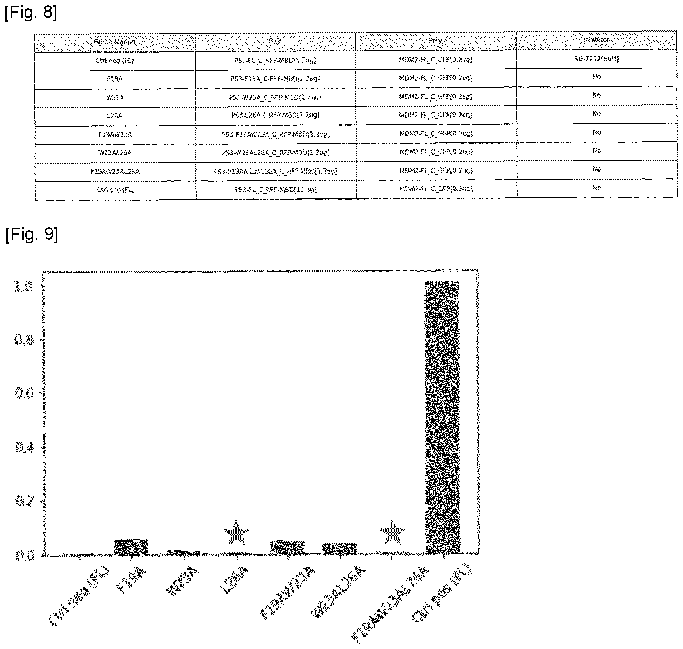

[Fig. 8] Conditions tested in the MT bench assay. U-2 OS cells were co-transfected with MDM2-FL-GFP and mutants of P53 and in some conditions were treated with the RG7112 small molecule.

[Fig. 9] Relative enrichment to the microtubules of MDM2 according to different p53 mutants. The stars indicate that MDM2 does not interact with p53 on the microtubules in these conditions, preventing the measure of a relative enrichment. The enrichment was normalized using a min-max normalization (with positive and negative controls as max and min) to allow a comparison between conditions.

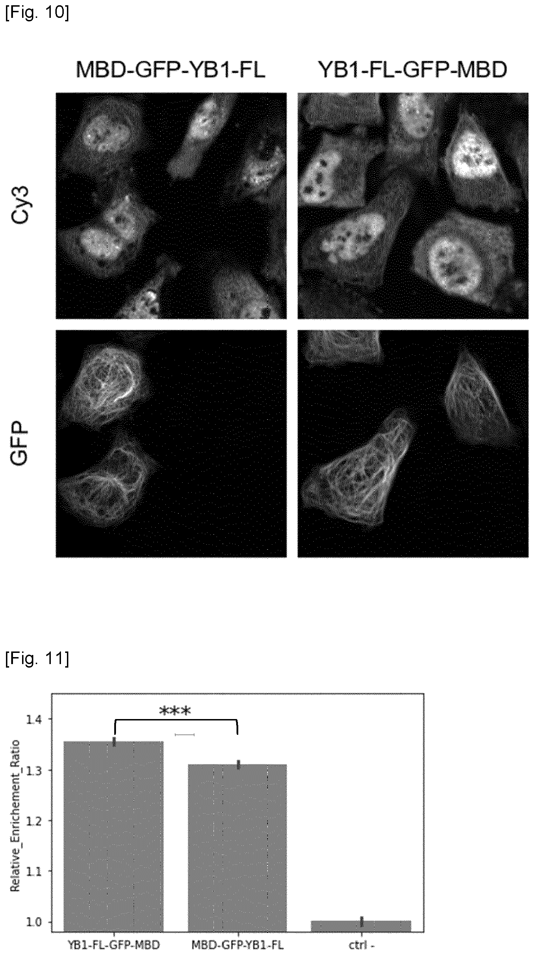

[Fig. 10] Cell images from the MTbench technology. Both constructs of YB1-FL fused to GFP used as baits and mRNA (Cy3 labelled) as prey. In both cases mRNA is localized at the microtubules.

[Fig. 11] Relative enrichment to the microtubules of mRNA in cells transfected with both YB1-FL constructs. The enrichments are calculated as defined in the material and methods. *** represents a p-value of 1.44e-5. Error bars represent the standard errors calculated from 16 replicates. The negative control corresponds to a condition where cells were transfected with GFP fused to MBD. There is no binding of RNA in this condition.

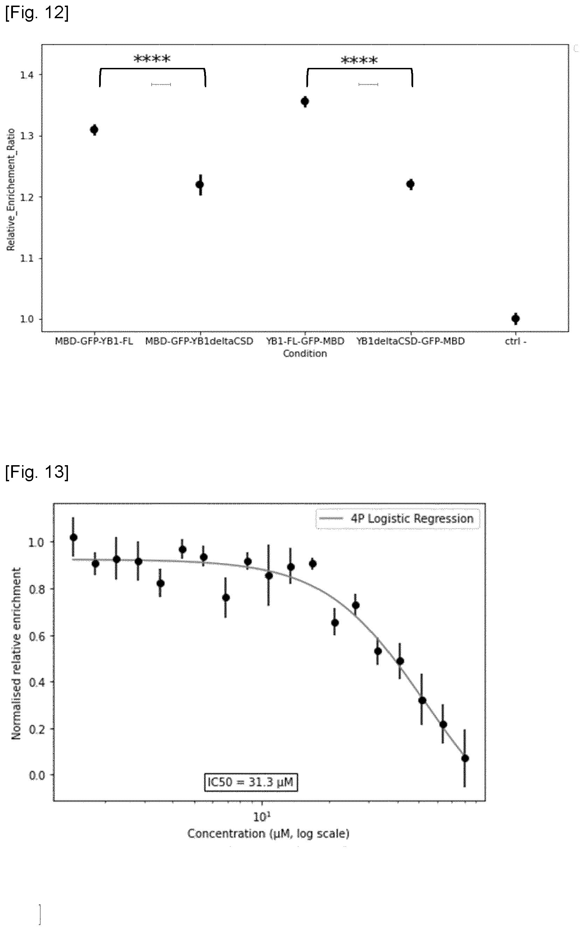

[Fig. 12] Relative enrichment to the microtubules of mRNA in cells expressing different DNA constructs. Enrichment values were obtained in 384-well plates. The negative control corresponds to a condition where cells were transfected with GFP fused to MBD. Error bars represent the standard errors calculated from 16 replicates. The p-values are the following:

- p-value (MBD-GFP-YB1-FL / MBD-GFP-YB1-deltaCSD) = 8.59e-7

- p-value (YB1-FL-GFP-MBD / YB1-deltaCSD-GFP-MBD) = 9.13e-20

[Fig. 13] Dose-response curve showing the inhibition of mRNA:YB1 interaction with increasing concentration of C8, allowing the determination of its activity (IC50) in a cellular environment.

**Definitions**

According to the invention, "evaluating the ability of a compound to disrupt or stabilize an interaction" refers that the method enables to determine if the said compound is able to have an effect on the interaction between one or more bait and one or more candidate prey. In particular, this method allows to determine if the said compound disrupt or stabilize this interaction.
According to the invention, a " bait " comprises at least one "bait moiety" and at least one "polymerized-tubulin binding moiety".
According to the invention, a "bait moiety " is a moiety which is prone to interact with one or more candidate prey(s) in the cell.
According to the invention, the expression "comprising " or "comprises " also includes "consisting " or "consisting of ".
A "polymerized-tubulin binding moiety " comprises a peptide, a protein, or a nucleoprotein which has the ability to bind specifically to polymerized tubulin. Preferably, the "polymerized-tubulin binding moiety" binds with a higher specificity to tubulin in its polymeric form than to tubulin in its non-polymeric form, which includes its monomeric form and/or its heterodimeric form (which corresponds to the alpha/beta tubulin heterodimer). Ideally, a polymerized-tubulin binding moiety binds mostly or even exclusively to polymerized-tubulin, which includes microtubules. The "polymerized-tubulin binding moiety " can be ideally chosen among known antibodies, microtubule-binding proteins, or fragments thereof, which includes (in a non-exhaustive manner) microtubule stabilizers and destabilizers, molecular motors, microtubule-severing proteins and end-tracking proteins such as plus-end tracking proteins.
Microtubules are a component of the cytoskeleton, found throughout the cytoplasm. Microtubules are part of a structural network (the "cytoskeleton ") within the cell's cytoplasm. The primary role of the microtubule cytoskeleton is mechanical support, although microtubules also take part in many other processes. Thus, microtubules are only part of the so-called "microtubule cytoskeleton ", because the latter further includes associated proteins, such as Microtubule-Associated Proteins (MAPs) along with other organizing structures such as the centrosomes.
"Polymerized tubulin " or "Polymerized-tubulin " refers exclusively to the assembly of monomeric tubulin, or alternatively of the assembly of heterodimers of tubulin, in a regular fashion and with a distinct polarity. Tubular polymers of tubulin can grow as long as 50 micrometers, with an average length of 25 $\mu$m, and are highly dynamic. The outer diameter of a microtubule is generally of about 24-25 nm while the inner diameter is of about 12 nm. They are found in eukaryotic cells and are formed by the polymerization of a dimer of two globular proteins, $\alpha$-tubulin and $\beta$-tubulin. Thus, the expression "polymerized tubulin " encompasses microtubules.

Thus, "microtubules " represent a particular rearrangement of "polymerized tubulin ", which occurs physiologically in eukaryotic cells, and which forms with additional partners the "microtubule cytoskeleton ". The physiological assembly of microtubules is generally described as comprising a first step of regulated assembly of alpha-tubulin and beta-tubulin heterodimers, which together form a polarized protofilament. Then, protofilaments are believed to assemble, as a cylinder, into the so-called microtubule. Thus, microtubules are generally described as polymers of dimers of $\alpha$- and $\beta$-tubulin, which are composed of 13 protofilaments assembled around a hollow core. However, it shall be noted that so-called microtubules with a different number of protofilaments have also been described in the Art, such as microtubules with 14 or 15 protofilaments. However, the physiological meaning of such variations, or "protofilament transitions ", remains unclear.

Tubulin refers to one of several members of a small family of globular proteins. The tubulin superfamily includes five distinct families, the alpha-, beta-, gamma-, delta-, and epsilon-tubulins and a sixth family (zeta-tubulin) which is present only in kinetoplastid protozoa. The most common members of the tubulin family are alpha-tubulin (a-tubulin) and beta-tubulin ($\beta$-tubulin), the proteins that make up microtubules. The end of the microtubule which corresponds to beta-tubulin is called the plus-end. The end of the microtubule which corresponds to alpha-tubulin is called the minus-end.

Thus, a "polymerized-tubulin binding moiety" of the invention may include an alpha-tubulin and/or a beta-tubulin binding moiety and/or combinations thereof, and binds preferably to tubulin in its polymeric form rather than its monomeric form or its heterodimeric form.

As known in the Art, the "heterodimeric form " of tubulin corresponds to the alpha/beta tubulin heterodimer. Thus, the polymeric form of tubulin also corresponds to a polymer of heterodimers, which also corresponds to more than one heterodimer of alpha/beta tubulin.

For reference, human alpha-tubulin is of sequence SEQ ID N°1.

For reference, human beta-tubulin is of sequence SEQ ID N°2

First, microtubules offer a large surface within the cell owing to their dimensions (25 nm in diameter and tenth of micrometers in length). If we consider one hundred 10 $\mu$m-long microtubules in typical mammalian cells like HeLa cells, the microtubule surface is larger than 30 $\mu$m2 and can even be larger in cells like neurons or muscle cells. Thus, such large surface is available for the binding of an enormous number of baits without saturation (if the bait requires an interacting surface as large as 10 nm2 on microtubules, virtually, more than 3 000 000 bait copies per cell can theoretically be anchored to microtubules). This is significantly higher than the typical number of over-expressed proteins in transfected mammalian cells (about 100 000 copies is already a large over-expression for most proteins).

A second advantage of using the microtubule surface lies in its dynamic behavior. Microtubules are intrinsically highly dynamics and alternate permanently between shortening and growing phases. This behavior allows to dissociate the bait from microtubules during the depolymerization phase and to bind to another microtubule or after repolymerization of the microtubule. During that interval of time, the bait moves away from microtubules and is then able to capture preys that are located in the bulk cytoplasm and not at the vicinity of microtubules.

It has been known for a long time that the detection of filamentous structures, such as "microtubule-like patterns " is highly sensitive and consists in extrapolating a straight line in the fluorescence image so that the signal can be detected out of a homogenously-distributed noise. This general principle is routinely used to image microtubules by fluorescence speckle microscopy (for a review, see Salmon & Waterman; How we discovered fluorescent speckle microscopy; Mol Biol Cell, 3940-2; 2011). However, this easy detection method would not be possible in more globular structure or other improperly defined substrate. Methods of the invention are also compatible with Real-time data analysis of the living cell fluorescent images, and thus can be implemented to better detect the bait bound to microtubules in living cells. The point is that the microtubules are moving with time due to their highly dynamical structures, which further improves their detection out of large fluorescent background for the detection in living cells.

"Microtubule-binding domain" refers to the one or more fragment(s) of a microtubule-binding protein that is/are responsible for its binding to polymerized tubulin, and in particular microtubules. A non-limiting list of microtubules-binding proteins is provided in Table 1 hereinbelow:

**Table 1**

| Function | Protein name or family |
|---|---|
| Microtubule stabilizers | MAP1 family |
| | Microtubule-associated protein 1A |

(continued)

| Function | Protein name or family |
|---|---|
| | Microtubule-associated protein 1B |
| | Microtubule associated proteins 1A/1B light chain 3A |
| | Microtubule associated proteins 1A/1B light chain 3B |
| | MAP RP/EB family |
| | Microtubule-associated protein RP/EB family member 1 |
| | Microtubule-associated protein RP/EB family member 2 |
| | Microtubule-associated protein RP/EB family member 3 |
| | WD repeat EMAP family |
| | Cytoskeleton-associated protein 5 (Disl/Tog) |
| | Ferritin heavy chain (Syncolin) |
| | MAP7 domain-containing protein 1 |
| | Microtubule-associated protein 2 |
| | Microtubule-associated protein 4 |
| | Microtubule-associated protein 7 |
| | Microtubule-associated protein tau |
| | STOP (MAP6) |
| Destabilizers | Kinesin-like protein KIF2A (Kinesin-2) |
| | Stathmin (Op18, prosolin, metablastin) |
| Molecular motors | Cytoplasmic dyneins & dynactin |
| | Kinesins |
| Severing | Katanin |
| | Spastin |
| Plus-end tracking proteins | Cytoplasmic linker proteins (CLIPs) : CLIP-170, CLIP-115, CLIP-190 |
| | CLIPs associating proteins (CLASPs) : CLASP 1, CLASP2, MAST/Orbit |
| | MCAK (mitotic centromere-associated kinesin) |
| | XMAP215, dCP224 |
| | EB/PR family proteins: EB-1, EB-2, EB-3, RP1, Bimlp, Mal3p |
| | Cytoplasmic linker proteins (CLIPs) : CLIP-170, CLIP-115, CLIP-190 |
| | Gephyrin |

Thus, the "polymerized-tubulin binding moiety " may be chosen among full-length microtubule-binding proteins, fragments of microtubule-binding proteins, or even isolated Microtubule-Binding Domains, which are known to bind specifically to polymerized tubulin, such as microtubules.

The "Microtubule-Binding Domain " (MBD) relates to the one or more fragment(s) of a microtubule-binding protein that is/are responsible for its binding to polymerized tubulin, and in particular microtubules.

According to the invention, "determining the occurrence of an interaction" refers to the identification that the said one or more protein bait and said one or more candidate prey make contact in the Microtubule-Binding system according to the invention. Said protein bait is bound to polymerized tubulin in the eukaryotic cell, thereby localizing said one or more candidate prey along said polymerized tubulin, thereby detecting said interaction if it occurs.

According to the invention, "Comparison of the occurrences of the interaction" refers to the identification of a potential change of interaction (disruption or stabilization) between steps b. and d., allowing to conclude whether the effect of the said compound is the disruption or the stabilization of the interaction. Other than disruption or stabilization, the method can also demonstrate the absence of change in the interaction

According to the invention, "disruption of the interaction" refers that the said one or more bait and one or more candidate prey are no longer interacting or that the interaction is weaker that it was before the step of contacting with the tested compound.

According to the invention, "stabilization of the interaction" refers that the said one or more bait and one or more candidate prey are interacting or that the interaction is stronger that it was before the step of contacting with the tested compound.

According to the invention, "quantification of the interaction" refers to the evaluation of the strength of the interaction occurring between the said one or more protein bait and said one or more candidate prey. This quantification allows the linking of the data obtained in steps b. and d. from the strength of the interaction between the said one or more bait and said one or more candidate prey, in order to conclude if the effect of the compound is to disrupts or stabilize the interaction, and to evaluate the strength of this disruption or stabilization.

According to the invention, "contacting the said eukaryotic cell with the said compound" refers to the introduction, in the medium where the eukaryotic cell is tested, of the said compound.

"IC50", or half maximal inhibitory concentration, is the most widely used and informative measure of a drug's efficacy. It indicates how much drug is needed to inhibit a biological process by half, thus providing a measure of potency of an antagonist drug in pharmacological research. In the case of this invention, the IC50 measure how much of the tested compound in needed to disrupts or stabilize the interaction between the said one or more protein bait and said one or more candidate prey.

According to the invention, a "physiological" condition may consist of an experimental set-up in which the eukaryotic cell is not in a situation of stress, or apoptosis, which includes an experimental set-up in which the eukaryotic cell is not in contact with a medium having a non-physiological pH, or salt condition.

According to the invention, a "Projection domain" refers to a domain found in MAPs such as MAP2 or Tau, and is involved in microtubule bundling and in determining the spacing between microtubules. The projection domains may also interact with other cytoskeletal structures. An example of a projection domain is the projection domain of the Tau protein, with the sequence SEQ ID NO: 3.

According to the invention, "Proteinopathy" refers to any disease or condition that results from the abnormal synthesis, folding, post-translational modification, or deposition of protein in cells or tissues. Examples of proteinopathies include, without limitation, Alzheimer's disease, Parkinson's disease, Huntington's disease and TDP-43 proteinopathies, such as ALS and FTLD.

According to the invention, "TDP-43 proteinopathies" refers to diseases characterized by the presence of abnormally phosphorylated, ubiquitinated, and cleaved DNA-binding protein TDP-43 in affected brain and spinal cord. Examples of TDP-43 proteinopathies include, without limitation, amyotrophic lateral sclerosis (ALS) and frontotemporal lobar degeneration (FTLD).

According to the invention, "Linker" refers to an unstructured domain, in particular an unstructured domain which allows a nm-long spacing between the polymerized-tubulin surface and the protein of interest. Examples of linkers are known in the art. In one embodiment, the linker is a projection domain of a microtubule-associated protein (MAP).

According to the invention, "High-Content Screening (HCS)" is an image-based method used in drug discovery to identify molecules (biologics or small molecules) that alter a phenotype using simultaneously several parameters of the cell as a readout (Fraietta and Gasparri, 2016; Mattiazzi Usaj et al., 2016).

According to the invention, "Compound" refers to any natural or chemical substance which may act on the interaction between the said one or more bait and said one or more candidate prey. This compound may be a known substance or came from a library of substance, based on *in vivo, in vitro,* or *in silico* data. According to the embodiment of this invention, the compound may be a medicament candidate, in case of the said one or more bait and said one or more candidate prey are characteristics of an interaction occurring in a specific pathology. In particular, said compound is a small molecule, a peptide, a protein, a RNA, or a DNA.

According to the invention, "Endogenous" refers to a bait and/or a prey coming from inside the organism of an individual.

According to the invention, the term "mutated" comprises deletion, truncation, addition or replacement of domains, amino-acids, or glycosylation.

## Detailed description of the invention

The invention relates to *in vitro* methods for evaluating the ability of a compound to disrupt or stabilize an interaction between one or more bait and one or more candidate prey in a eukaryotic cell.

In particular, said *in vitro* method for evaluating the ability of a compound to disrupt or stabilize an interaction between one or more bait and one or more candidate prey in a eukaryotic cell, comprises the steps of:

a. providing a eukaryotic cell expressing (i) one or more bait, and (ii) one or more candidate prey, wherein said bait comprises a bait moiety and a polymerized tubulin-binding moiety;

b. determining the occurrence of an interaction between said one or more bait and said one or more candidate prey in the eukaryotic cell, wherein said bait is bound to polymerized tubulin in the eukaryotic cell, thereby localizing said one or more candidate prey along said polymerized tubulin, thereby detecting said interaction;

c. contacting the said eukaryotic cell with the said compound;

d. determining the occurrence of the interaction between said one or more bait and said one or more candidate prey in the eukaryotic cell as in step b. and quantify this interaction;

e. Comparison of the occurrences of the interaction made in step b. and in. step d.;

f. Conclude whether the effect of the said compound is the disruption or the stabilization of the interaction between said one or more bait and one or more candidate prey.

**Methods and uses for detecting an interaction between bait(s) and candidate prey(s), for steps a., b., and d.**

According to one embodiment, said " bait" comprises at least one "bait moiety" and at least one "polymerized-tubulin binding moiety" which are not from a same naturally-occurring entity.

Baits can be brought to microtubules using a fused polymerized-tubulin binding moiety, such as Microtubule-Associated Proteins (MAPs) including tau (NCBI Reference Sequence: NP_005901.2 or SEQ ID NO: 14), or Microtubule-Binding Domains (MBD).

According to one embodiment, the anchoring of the bait is due to the repetition of Microtubule-Binding Domains generally present in MAPs. These repeats have a high affinity toward microtubules compared to free tubulin, the building block of microtubules, and only a weak portion will be free in the cell cytoplasm using this method.

When the prey interacts with the bait brought to microtubules, its relocation on microtubule surface allows its detection via the appearance of microtubular structures in the prey's fluorescent image.

It has been known for a long time that the detection of filamentous structures, such as "microtubule-like patterns " is highly sensitive and consists in extrapolating a straight line in the fluorescence image so that the signal can be detected out of a homogenously-distributed noise. This general principle is routinely used to image microtubules by fluorescence speckle microscopy (for a review, see Salmon & Waterman; How we discovered fluorescent speckle microscopy; Mol Biol Cell, 3940-2; 2011).

However, this easy detection method would not be possible in more globular structure or other improperly defined substrate. Methods of the invention are also compatible with Real-time data analysis of the living cell fluorescent images, and thus can be implemented to better detect the bait bound to microtubules in living cells. The point is that the microtubules are moving with time due to their highly dynamical structures, which further improves their detection out of large fluorescent background for the detection in living cells.

For all these reasons, the choice of microtubules is highly relevant, even compared to other kinds of filaments. As the interaction between the bait and the prey can be observed in real time, the impact of the tested compound can be followed.

Methods and uses of the invention have been further validated, as shown from the examples. Briefly, a plasmid has been designed to direct the expression of a known protein (bait) fused to the tau protein via its N-terminal or C-terminal domain (end of the projection domain of tau) in mammalian cells.

After being fused to tau, two baits were successfully brought to microtubules: P53 (tumor protein 53; Accession Number: BAC16799) a transcription factor and YB-1 (nuclease-sensitive element-binding protein 1 or Y-box binding protein; Accession Number : NP_004550.2), a mRNA-binding protein, have both been easily detected along microtubules by optical microscopy in HeLa cells (human cancer cells respectively).

After being fused to tau, two baits were successfully brought to microtubules: P53 (tumor protein 53; Accession Number : BAC16799) a transcription factor and YB-1 (nuclease-sensitive element-binding protein 1 or Y-box binding protein; Accession Number : NP_004550.2), a mRNA-binding protein, have both been easily detected along microtubules by optical microscopy in cellules U2-OS (epithelial morphology cell line derived from a moderately differentiated sarcoma).

The "bait moiety " and the candidate prey may be identical or different, and are preferably selected in a group comprising (or consisting in) a protein or a nucleic acid, which includes nucleoproteins, and deoxyribonucleic acid.

In a non-limitative manner, a "bait moiety " and/or a "candidate prey " includes any molecule of interest, particularly proteins of interest, such as antibodies, nucleic-acid binding proteins and various other bait receptor proteins or peptides, notably those of biological relevance, including those of diagnostic and pharmacological relevance. It also includes nucleic acids, such as messenger RNAs, coding and noncoding RNAs, transfer RNAs, ribosomal RNAs, interfering RNAs or silencing RNAs. It also includes deoxyribonucleic acid of any type, such as antisense DNA, circular DNA, complementary DNA, spacer DNA, heterologous DNA, hybrid DNA, homoduplex DNA, linking DNA, mobile DNA (transposon), ribosomal DNA, satellite DNA, single-stranded DNA, doublestranded DNA, superhelical DNA, or triple-stranded DNA.

In the method according to the invention, the said RNA, protein or DNA is endogenous.

The interaction between the bait and the prey is assessed due to the interaction of the bait to polymerized tubulin by a polymerized-tubulin binding moiety. Thus, it is preferable that said bait moiety does not interact or colocalize at all with polymerized tubulin, or in a limited manner, when compared to the same moiety conjugated to the polymerized tubulin binding moiety under reference and/or physiological conditions.

Bait moieties which are particularly considered are bait moieties of which the distribution is diffuse in the cytoplasm of the eukaryotic cell under reference and/or physiological conditions.

According to some embodiments, the "bait moiety " is a moiety that does not interact, or co-localize, with polymerized tubulin (or microtubules) in the eukaryotic cell under reference or physiological conditions.

According to one exemplary embodiment a "bait moiety " is a nucleic-acid binding moiety, such as a nucleic-acid binding protein, and a "candidate prey " is a nucleic acid, such as a messenger RNA.

According to another embodiment, a "bait moiety " is an antibody or a fragment thereof, and a "candidate prey " is a protein or a peptide that is susceptible to bind to said antibody.

According to another embodiment, a "bait moiety " is a nucleic acid, such as a messenger RNA, and a "candidate prey " is a nucleic-acid binding moiety, such as a nucleic-acid binding protein.

A "candidate prey " can be a native or modified, homologous or heterologous candidate prey.

Although "Microtubule-Associated Proteins " (MAP) may be also polymerized-tubulin binding proteins and/or Microtubule-binding proteins, the expression "Microtubule-Binding Domain " (MBD) refers to a domain that is able to specifically and bind directly to microtubules. For the same reasons, a "polymerized-tubulin binding moiety " will refer to a moiety that binds specifically and directly to polymerized-tubulin.

Thus, a MBD may comprise all the possible sequences of amino acids that lead to the binding of the microtubule-binding protein to microtubules.

For reference, a Microtubule-Binding Domain of the invention may be derived from the Tau protein, such as the Tau isoform 2 (Accession Number: NP_005901.2), which includes sequence SEQ ID N°4, which includes sequences SEQ ID N°5 to 8.

A Microtubule-Binding Domain of the invention may also be derived from the MAP1A protein (Accession Number: NP_002364), which includes sequence SEQ ID N°9.

A Microtubule-Binding Domain of the invention may also be derived from the MAP2 protein (Accession Number: NP_002365), which includes sequence SEQ ID N°10.

A Microtubule-Binding Domain of the invention may also be derived from the MAP4 protein (Accession Number: AAA67361), which includes sequence SEQ ID N°11.

A Microtubule-Binding Domain of the invention may also be derived from the MAP6 protein (Accession Number: NP_149052), which includes sequence SEQ ID N°12.

A Microtubule-Binding Domain of the invention may also be derived from the EB1 protein (Accession Number: NP_036457), which includes sequence SEQ ID N°13.

Thus, a Microtubule-Binding Domain of the invention may be selected in a group comprising or consisting of: Tau of sequence SEQ ID N°4 and SEQ ID N°5 to 8, MAP1A of sequence SEQ ID N°9, MAP2 of sequence SEQ ID N°10, MAP4 of sequence SEQ ID N°11, MAP6 of sequence SEQ ID N°12, EB-1 of sequence SEQ ID N°13 and/or any Microtubule-Binding Domain that is derived from Microtubule-Associated proteins, and fragments, and combinations thereof.

Methods for identifying polymerized-tubulin binding moieties and/or microtubule-binding domains in a protein have already been reported in the Art. See for reference: Cravchik et al.; Identification of a novel microtubule-binding domain in microtubule-associated protein 1A (MAP1A). J Cell Sci, 107 (Pt 3),661-72,1994.

According to one embodiment, said bait comprises:

- a polymerized tubulin-binding moiety comprising one or more Microtubule- Binding Domain (MBD), and

- a bait moiety.

According to one embodiment, said bait comprises one Linker (L) region located between the polymerized-tubulin binding moiety and the bait moiety.

According to one embodiment, the Microtubule-Binding Domain is selected in a group consisting of: Tau of sequence SEQ ID N°4 and SEQ ID N°5 to 8, MAP1A of sequence SEQ ID N°9, MAP2 of sequence SEQ ID N°10, MAP4 of sequence SEQ ID N°11, MAP6 of sequence SEQ ID N°12, EB-1 of sequence SEQ ID N°13 and/or any Microtubule-Binding Domain that is derived from Microtubule-Associated proteins, and fragments, and combinations thereof.

According to one embodiment, the candidate prey comprises a fluorescent protein.

According to an embodiment, the method according to the invention comprise a c'. step between steps c. and d. of controlling that the compound is well entered into the cell. This optional step allows to confirm that the tested compound may have act on the interaction between the bait and the prey. Cell treatments with various compounds involve the use of these molecules diluted in a solvent, such as DMSO, and the duration of the treatment can range from a few minutes to several hours, or even days. This flexibility in treatment duration enables researchers to evaluate the effects of compounds on cellular processes.

According to an embodiment, step b. and d. also comprise the quantification of the interaction between said one or more bait and said one or more candidate prey, allowing a comparison between the interaction before or after contact with the tested compound, and allowing to determine the strength of disruption or stabilization of the compound.

According to one embodiment, the cell in step b. is a fixed cell or a living cell.

The method provided in steps a. b. and d. allow to rapidly identify the changes of interactions in living and fixed eukaryotic cells, such as mammalian cells.

The nucleic acid sequence coding for the bait is inserted in a plasmid to be fused to a given microtubule-binding domain. Then, a screening can be performed with antibodies directed against the potential endogenous partners in fixed cells or with complementary oligonucleotide to detect nucleic acid interacting-partner.

In both living and fixed cells, classical GFP-tagged plasmid encoding for a given set of preys relevant to the investigated interactions can be used to screen for potential partners of a given bait and compared with control (only tau-GFP protein for example).

High throughput analysis of protein and nucleic acid interactomes in 96-, -384, -1536-well plates in fixed or living cells is also possible.

Steps a. b. and d. are able to generate a "microtubule-like pattern" or a "polymerized tubulin-like pattern" if (i) the candidate prey is able to interact with the bait, and if (ii) the bait is simultaneously bound to microtubules or polymerized-tubulin respectively in the eukaryotic cell.

Thus, the detection of said "microtubule-like pattern" or "polymerized tubulin-like pattern" is indicative of an interaction between said one or more bait and said one or more candidate prey in the eukaryotic cell.

In particular, the detection of the candidate prey in the eukaryotic cell is able to generate a "microtubule-like pattern" or "polymerized tubulin-like pattern" if (i) the candidate prey is able to interact with the bait moiety, and if (ii) the polymerized-tubulin binding moiety is simultaneously bound to microtubules or polymerized tubulin respectively in the eukaryotic cell.

Thus, according to said embodiment, the detection of said "microtubule-like pattern" or "polymerized tubulin-like pattern" is also indicative of an interaction between said one or more bait moiety and said one or more candidate prey in the eukaryotic cell.

According to one embodiment, said one or more candidate prey and/or said one or more bait is a ribonucleic acid.

According to one embodiment, said one or more candidate prey and/or said one or more bait is a deoxyribonucleic acid.

According to one embodiment, said one or more candidate prey and/or said one or more bait is a protein.

In summary, the combinations of said one or more candidate prey and said one or more bait can be as follows:

i. said one or more bait: at least a protein / said one or more candidate prey: at least a protein

ii. said one or more bait: at least a protein / said one or more candidate prey: at least a ribonucleic acid

iii. said one or more bait: at least a protein / said one or more candidate prey: at least a deoxyribonucleic acid

iv. said one or more bait: at least a ribonucleic acid / said one or more candidate prey: at least a protein

v. said one or more bait: at least a ribonucleic acid / said one or more candidate prey: at least a ribonucleic acid

vi. said one or more bait: at least a ribonucleic acid / said one or more candidate prey: at least a deoxyribonucleic acid

vii. said one or more bait: at least a deoxyribonucleic acid / said one or more candidate prey: at least a protein

viii. said one or more bait: at least a deoxyribonucleic acid / said one or more candidate prey: at least a ribonucleic acid

ix. said one or more bait: at least a deoxyribonucleic acid / said one or more candidate prey: at least a deoxyribonucleic acid

According to a preferred embodiment, said one or more bait is a protein.

According to a preferred embodiment, said one or more candidate prey is a protein or a ribonucleic acid.

According to an embodiment of the invention, in case the prey is a protein, said one or more candidate prey may comprise a wild-type protein and a mutated version of the same protein.

According to one embodiment, the wild-type and mutated forms of the proteins of interest comprise the sequence of the wild-type form or the mutated form of the protein of interest, a fluorescent tag, a linker and one or more microtubule-binding domain(s). Preferably, the one or more microtubule-binding domain(s) is/are selected from the group comprising or consisting of: Tau of sequence SEQ ID NO: 4 and SEQ ID NO: 5 to 8, MAP1A of sequence SEQ ID NO: 9, MAP2 of sequence SEQ ID NO: 10, MAP4 of sequence SEQ ID NO: 11, MAP6 of sequence SEQ ID NO: 12, EB-1 of sequence SEQ ID NO: 13 and/or any microtubule-binding domain that is derived from microtubule-associated proteins, and fragments, and combinations thereof.

Methods and uses of the invention are suitable for evaluating the ability of a compound to disrupt or stabilize an interaction between one or more bait and one or more candidate prey in a eukaryotic cell, which includes one bait and one candidate prey, one bait and more than one candidate prey, and more than one bait and one candidate prey.

Methods of the invention are also suitable for evaluating the ability of a compound to disrupt or stabilize an interaction between a plurality of baits and a plurality of candidate preys.

According to another embodiment, the method is for evaluating the ability of a compound to disrupt or stabilize an interaction between one bait and one candidate prey in the eukaryotic cell.

According to the invention, a "eukaryotic cell" includes any eukaryotic cell that is susceptible to contain and/or express polymerized-tubulin, such as microtubules.

According to a particular embodiment, the eukaryotic cell is selected in a group comprising primary cells, stable cell lines, stem cells and Induced Pluripotent Stem cells (IPS). For example, the eukaryotic cell may be selected in a list comprising, or consisting of mammalian cells and insect cells.

In particular, the eukaryotic cell may be selected in a list comprising, or consisting of: HEK cells, NR cells, U2 -OS cells and HeLa cells, in particular HeLa cells.

The cell in step b. can be a fixed cell or a living cell.

When the cell in step b. is a living cell, it is preferred that the bait and/or the candidate prey comprise a detectable tag such as a fluorescence tag.

The use of a living cell in step b. allows for detection of the candidate prey along dynamical polymers of tubulin, such as microtubules, when interacting with the bait.

When the cell in step b. is a fixed cell, the method requires an additional step of fixation, prior to step b.. Protocols for obtaining fixed cells are well known in the Art.

In any case, microtubules appear as bright lines on fluorescence images, which renders the binding of the preys on them easily detectable by probing for instance the appearance of long bright lines using the prey's fluorescence signal, which leads to a characterizing "microtubule-like pattern".

The candidate prey may be a modified or unmodified candidate prey. The candidate prey may also be a homologous or heterologous candidate prey.

The bait and/or the candidate prey may also comprise a detectable moiety. In particular, the detectable moiety may be a fluorescent protein.

The detectable moiety may be selected in a group comprising: GFP, YFP, XFP, RFP, CFP, DsRED, mCherry, Luciferase, Cyanine dyes such as Cy2 or Cy3 or Cy5, fluorescein, rhodamine, and Alexa fluor dyes.

In particular, the fluorescent protein can be selected in a group comprising: GFP, YFP, XFP, RFP, CFP, DsRED, and mCherry.

Fluorescent proteins are widely known in the Art and may be found, for instance, in Shaner & Steinbach & Tsien (A guide to choosing fluorescent proteins; Nat Methods; 2(12):905-9; 2005).

Said bait is bound to polymerized tubulin in the eukaryotic cell, thereby localizing said one or more candidate prey along said polymerized tubulin, thereby detecting said interaction.

According to another embodiment, the invention relates to a method as defined above, wherein in steps b. and d., the determination of the occurrence of the said prey in the eukaryotic cell, is made with a detection method selected in a group comprising: binding to an antibody, hybridization with a nucleic acid, and/or fluorescence measurement.

For instance, steps b. and d. of determining the occurrence of an interaction can be achieved using videomicroscopy.

Steps b. and d. of determining the occurrence of an interaction can also be achieved using a computer-assisted recognition method, such as a computer-assisted recognition method taught in Altinok et al. (Activity analysis in microtubule videos by mixture of hidden Markov models; Computer Vision and Pattern Recognition, IEEE Computer Society Conference, 2, 1662-1669; 2006).

In addition, the invention relates to a method as defined above, comprising before steps b. and d., at least one step of depolymerizing cellular tubulin using a Microtubule-Depolymerizing drug or cold exposure.

Microtubule-disrupting, Microtubule-depolymerizing or Microtubule-disassembling drugs, such as Nocodazole, Vinblastine, Vincristine, Colchicine, Colcemid, Podophyllotoxin, Rizhoxin or Vinorelbine, can also be used for that purpose.

Cold exposure is known in the Art and generally relates to a step of depolymeryzing microtubules by exposing them to low temperature. Protocols which relate to cold exposure are known in the Art, and for instance are taught in Ochoa et al. Cold exposure reveals two populations of microtubules in pulmonary endothelia; Am. J. physiol. Lung Cell. Mol. Physiol; 300:L132-L138; 2011).

Steps of polymerization and depolymerization as defined above may be advantageously repeated over time, leading to a succession of alternating phases of association and dissociation of the bait over time.

Thus, for the *in vitro* method according to the invention, the steps may be arranged as follow:

a. providing an eukaryotic cell expressing (i) one or more bait, and (ii) one or more candidate prey, wherein said bait comprises a bait moiety and a polymerized-tubulin binding moiety,

optionally depolymerizing cellular tubulin using a Microtubule-Depolymerizing drug or cold exposure,

b. determining the occurrence of a first interaction between said one or more bait and said one or more candidate prey in the eukaryotic cell, wherein said bait is bound to polymerized tubulin in the eukaryotic cell,
optionally depolymerizing cellular tubulin using a Microtubule-Depolymerizing drug or cold exposure, and

b'. determining the occurrence of a second interaction between said one or more bait and said one or more candidate prey in the eukaryotic cell, wherein said bait is bound to polymerized tubulin in the eukaryotic cell.

c. contacting the said eukaryotic cell with the said compound;
optionally depolymerizing cellular tubulin using a Microtubule-Depolymerizing drug or cold exposure, and

d. determining the occurrence of a first interaction between said one or more bait and said one or more candidate prey in the eukaryotic cell as in step b. and quantify this interaction;
optionally depolymerizing cellular tubulin using a Microtubule-Depolymerizing drug or cold exposure, and

d'. determining the occurrence of a second interaction between said one or more bait and said one or more candidate prey in the eukaryotic cell as in step b. and quantify this interaction;

In step b., said bait is bound to polymerized tubulin in the eukaryotic cell, thereby localizing said one or more candidate prey along said polymerized tubulin, thereby detecting said first interaction.

In step b'., said bait is bound to polymerized tubulin in the eukaryotic cell, thereby localizing said one or more candidate prey along said polymerized tubulin, thereby detecting said second interaction.

In step d., said bait is bound to polymerized tubulin in the eukaryotic cell, thereby localizing said one or more candidate prey along said polymerized tubulin, thereby detecting said first interaction.

In step d'., said bait is bound to polymerized tubulin in the eukaryotic cell, thereby localizing said one or more candidate prey along said polymerized tubulin, thereby detecting said second interaction.

Of course, steps of depolymerization may be repeated over time.

According to said methods, it is possible to detect not only interactions between baits and preys, but also the dynamics of polymerized-tubulin in the eukaryotic cell.

Because the bait and the prey are able to interact in a living environment, it is also an object of the invention to allow not only the impact of a compound on an interaction between the bait and the prey, but also to detect post-translational modifications that may occur in the eukaryotic cell, and can affect the efficacy of the compound. Thus, methods of the invention may further include a step of detecting the occurrence of a post-translational modification, for instance of the bait moiety and/or of the candidate prey. Post-translational modifications are well known in the Art and include, for instance, modifications like phosphorylation events.

Thus, this method is also efficient for identifying, in real-time, post-translational events such as phosphorylation that modify bait-prey interactions, using for instance video microscopy, as detailed in the examples for eIF2B-eIF2A interactions.

The invention also relates to the use of a polymerized-tubulin binding moiety, as a tool for evaluating the ability of a compound to disrupt or stabilize an interaction between one or more protein bait and one or more candidate prey in a eukaryotic cell.

Said bait is bound to polymerized tubulin in the eukaryotic cell, thereby localizing said one or more candidate prey along said polymerized tubulin, thereby detecting the ability of a compound to disrupt or stabilize an interaction between one or more protein bait and one or more candidate prey.

In particular, the invention relates to the use of a polymerized-tubulin binding moiety fused to one or more bait, as a tool for evaluating the ability of a compound to disrupt or stabilize an interaction between one or more protein bait and one or more candidate prey in a eukaryotic cell. Said bait is bound to polymerized tubulin in the eukaryotic cell, thereby localizing said one or more candidate prey along said polymerized tubulin, thereby evaluating the ability of a compound to disrupt or stabilize an interaction between one or more protein bait and one or more candidate prey.

More particularly, the invention relates to the use of at least one Microtubule-Binding Domain fused to one or more bait, as a tool for evaluating the ability of a compound to disrupt or stabilize an interaction between one or more protein bait and one or more candidate prey in a eukaryotic cell. Said bait is bound to polymerized tubulin in the eukaryotic cell, thereby localizing said one or more candidate prey along said polymerized tubulin, thereby evaluating the ability of a compound to disrupt or stabilize an interaction between one or more protein bait and one or more candidate prey.

According to said embodiment, the invention also relates to the use of a plurality of Microtubule-Binding Domains, such as tandems of Microtubule-Binding Domains, fused to one or more bait, as a tool for evaluating the ability of a compound to disrupt or stabilize an interaction between one or more bait and one or more candidate prey in a eukaryotic cell.

Preferably, the polymerized-tubulin binding moiety and/or the Microtubule-Binding Domain may be used in the form of a bait as disclosed further herebelow.

**Bait**

Herein is also described a bait, that is suitable for the methods and uses as defined above.

In particular, herein is described a bait comprising (i) a polymerized-tubulin binding moiety comprising one or more Microtubule-Binding Domain (MBD), and (ii) a bait moiety.

According to some embodiments, the "bait moiety " does not interact, or co-localize, with polymerized tubulin or microtubules in the eukaryotic cell.

According to some embodiments, the bait moiety may consist of antibodies or fragments thereof, or nucleic-acid binding proteins. Thus, according to some embodiments, the protein bait comprises:

- a polymerized tubulin-binding moiety comprising one or more Microtubule-Binding Domain(s), and

- a bait moiety that does not interact with polymerized tubulin in the eukaryotic cell.

According to some embodiments, the bait moiety may consist of antibodies or fragments thereof, or nucleic-acid binding proteins. Thus, according to some embodiments, the protein bait comprises:

- a polymerized tubulin-binding moiety comprising one or more Microtubule-Binding Domain(s), and

- a bait moiety consisting of antibodies or fragments thereof or nucleic-acid binding proteins.

A barrier for interaction can be a reduction of the accessibility of the bait when fused to a polymerized tubulin-binding moiety due to the proximity of the microtubule surface. To maximize the bait's accessibility to the prey, the bait can be attached to a projection domain, which is preferably an unstructured tail allowing a nm-long spacing between the microtubule or the polymerized-tubulin surface and the bait.

Thus, according to a particular embodiment, a bait suitable for the methods and uses of the invention comprises a polymerized tubulin-binding moiety comprising one or more Microtubule-Binding Domain (MBD), and a bait moiety.

Advantageously, the Microtubule-Binding Domains (MBD) are selected among a group comprising: Tau of sequence SEQ ID N°4, MAP1A of sequence SEQ ID N°9, MAP2 of sequence SEQ ID N°10, MAP4 of sequence SEQ ID N°11, MAP6 of sequence SEQ ID N°12, EB-1 of sequence SEQ ID N°13, or any other Microtubule-Binding Domain that is derived from Microtubule-Associated proteins, and combinations thereof.

According to a preferred embodiment, said one or more bait is a protein.

According to some embodiments, the protein bait comprises: a polymerized tubulin-binding moiety comprising one or more Microtubule-Binding Domain(s), selected in a group consisting of: Tau of sequence SEQ ID N°4 and SEQ ID N°5 to 8, MAP1A of sequence SEQ ID N°9, MAP2 of sequence SEQ ID N°10, MAP4 of sequence SEQ ID N°11, EB-1 of sequence SEQ ID N°13 and/or fragments, and combinations thereof; and a bait moiety.

According to some embodiments, the protein bait comprises a polymerized tubulin-binding moiety comprising one or more Microtubule-Binding Domain(s), selected in a group consisting of: Tau of sequence SEQ ID N°4 and SEQ ID N°5 to 8, MAP1A of sequence SEQ ID N°9, MAP2 of sequence SEQ ID N°10, MAP4 of sequence SEQ ID N°11, EB-1 of sequence SEQ ID N°13 and/or fragments, and combinations thereof; and a bait moiety that does not interact with polymerized tubulin in the eukaryotic cell.

According to some embodiments, the protein bait comprises: a polymerized tubulin-binding moiety comprising one or more Microtubule-Binding Domain(s), selected in a group consisting of: Tau of sequence SEQ ID N°4 and SEQ ID N°5 to 8, MAP1A of sequence SEQ ID N°9, MAP2 of sequence SEQ ID N°10, MAP4 of sequence SEQ ID N°11, EB-1 of sequence SEQ ID N°13 and/or fragments, and combinations thereof; and a bait moiety consisting of antibodies or fragments thereof or nucleic-acid binding proteins.

Accordingly, the bait may further comprise one Linker (L) region located between the polymerized tubulin-binding moiety and the bait moiety.

A Linker (L) region is generally an unstructured domain, in particular an unstructured domain which allows a nm-long spacing between the polymerized-tubulin surface and the bait, which is critical to increase the accessibility of the bait to the prey. Examples of Linker regions are known in the Art.

Projection domains are found in Microtubule-Associated Proteins (MAPs) such as MAP2 or Tau, and are involved in microtubule bundling and in determining the spacing between microtubules. They may also interact with other cytoskeletal structures.

Preferably, in order to maximize the bait's accessibility to the prey, the bait is attached to a Linker region which is a projection domain from a MAP, such as Tau, or a fragment thereof.

Thus, according to a preferred embodiment, the protein bait comprises (i) a polymerized-tubulin binding moiety comprising one or more Microtubule-Binding Domain (MBD), (ii) a projection domain and (ii) a bait moiety.

A "projection domain " suitable for the methods and uses of the invention may comprise or consist of a N-terminal fragment of Tau.

According to a particular embodiment, a "projection domain " is the Tau projection domain of sequence SEQ ID N°3, or a fragment thereof.

The Linker region, or projection domain, can be of varying length, which includes any region or domain as defined above of from 1 to 150 amino acids in length, which includes 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 and 150 amino acids in length.

When the bait comprises (i) a polymerized-tubulin binding moiety comprising one or more Microtubule-Binding Domains and (ii) a projection domain, it is preferable that both parts are part of the same protein, in particular the same Microtubule-Associated Protein, such as Tau.

The bait moiety may or may not comprise a detectable moiety, such a fluorescent protein.

Advantageously, the bait may further comprise any detectable moiety as defined above, at its N-terminal or C-terminal part, such as a fluorescent label that is detectable using fluorescence microscopy.

In a non-limitative manner, the detectable moiety may be a fluorescent protein, such as a protein selected in a group comprising: GFP, YFP, XFP, RFP, CFP, DsRED, and mCherry.

**Vectors and cell lines**

Herein is also described a vector containing an expression cassette suitable for expressing a bait according to the uses and methods of the invention.

In particular, the invention relates to a vector containing an expression cassette suitable for expressing a protein bait of the invention.

In particular, the invention relates to a vector containing an expression cassette suitable for expressing a ribonucleic acid bait of the invention.

In particular, the invention relates to a vector containing an expression cassette suitable for expressing a deoxyribonucleic acid bait of the invention.

In particular, the invention relates to a vector containing an expression cassette suitable for expressing a protein prey of the invention.

In particular, the invention relates to a vector containing an expression cassette suitable for expressing a ribonucleic acid prey of the invention.

In particular, the invention relates to a vector containing an expression cassette suitable for expressing a deoxyribonucleic acid prey of the invention.

Thus, a vector suitable for the uses and methods of the invention is suitable for expressing a bait, which includes vectors comprising:

- an expression cassette coding for a bait moiety fused to a polymerized-tubulin binding moiety comprising one or more Microtubule-Binding Domain (MBD), or alternatively,

- an expression cassette coding for a polymerized-tubulin binding moiety comprising one or more Microtubule-Binding Domain (MBD) and which is directly suitable for cloning at least one bait moiety as a fused bait.

An expression cassette is directly suitable for cloning at least one bait moiety as a fused protein bait, according to standard cloning protocols, if it is suitable for the introduction of an insert coding for said bait moiety in frame with said polymerized-tubulin binding moiety. Standard cloning protocols include protocols comprising a step of digestion with restriction enzymes, and/or site-specific recombination.

A vector may include one, or more than one, selectable marker. A selectable marker is a marker gene introduced into a cell, especially a bacterium or cells in culture, that confers a trait suitable for artificial selection.

According to preferred embodiments, the expression cassette is coding for a polymerized-tubulin binding moiety comprising one or more Microtubule-Binding Domain (MBD) which are derived from a Microtubule-Associated Protein of the invention.

According to a most preferred embodiment, the expression cassette is coding for a polymerized-tubulin binding moiety comprising one or more Microtubule-Binding Domain (MBD) which are derived from the protein Tau of sequence SEQ ID N°14. Vectors suitable for the uses and methods of the invention, may comprise:

- an expression cassette coding for a protein library for said bait moiety fused to a polymerized-tubulin binding moiety comprising one or more Microtubule-Binding Domain (MBD), or alternatively,

- an expression cassette coding for a polymerized-tubulin binding moiety comprising one or more Microtubule-Binding Domain (MBD) and which is directly suitable for cloning a nucleic acid coding for a protein library for said bait moiety as a fused protein bait.

Examples of vectors which are suitable for expression in eukaryotic cells include the Gateway© pEF-Dest51 plasmid.

Herein are also described cell lines that are transfected with a vector as previously described, and/or that stably express a bait suitable for the methods and uses of the invention.

Herein are further described to kits for evaluating the ability of a compound to disrupt or stabilize an interaction between said one or more bait and one or more prey in a cell, comprising a bait, a vector, and/or a cell line suitable for the methods and uses of the invention.

Herein is also described a kit for evaluating the ability of a compound to disrupt or stabilize an interaction between said one or more bait and one or more prey in a cell, comprising a bait, a vector, and/or a cell line suitable for the methods and uses of the invention, and combinations thereof.

Herein is also described a kit as defined above, comprising a nucleic acid library coding for the said bait or the said prey, and/or a protein library comprising said bait or the said prey, and/or a deoxyribonucleic acid library coding for the said bait and/or a reagent suitable for detecting the occurrence of said bait or said prey in the said cell, and combinations thereof.

Herein is also described a kit for evaluating the ability of a compound to disrupt or stabilize an interaction between said one or more bait and one or more prey in a eukaryotic cell, comprising

- a vector of the invention, and/or a cell line of the invention,

- optionally, a protein library for one or more bait moiety,

- optionally, a reagent suitable for detecting the occurrence of said protein bait and/or said candidate prey in a eukaryotic cell.

Herein is also described a kit for evaluating the ability of a compound to disrupt or stabilize an interaction between said one or more bait and one or more prey in a eukaryotic cell, comprising:

- a vector of the invention, and/or a cell line of the invention,

- a nucleic acid library or a protein library coding for one or more bait moiety,

- optionally, a reagent suitable for detecting the occurrence of said protein bait and/or said candidate prey in a eukaryotic cell.

According to one embodiment of the invention, steps a. and b. are replicate before step c. with different vector constructs of said one or more bait and said one or more candidate prey, in order to determine the occurrence of the interactions between said one or more bait and said one or more candidate prey in the eukaryotic cell, and choose the right combination to be evaluate in the following steps to be closer to what happens in vivo.

In a preferred embodiment, a vector library (pDEST plasmid) is generated combinatorically, specific to the method according to the invention and exhaustive in order to characterize the modes of interaction between PPI or RPI, whatever the system.

In a combinatorial manner, pDEST vectors are generated in order to consider all possible combinations of fusions with respect to given fluorophores.

In the case of a PPI interaction between protein A (bait) and protein B (prey), the following pDEST vectors can be used, where pDON can be protein A (A_pDON) and protein B (B_pDON):

4 pDEST vectors in Nter :

a. Bait: A_pDON-GFP-MBD (SEQ ID N° 14)

b. Bait: A_pDON-RFP- MBD (SEQ ID N° 14)

c. Prey: B_pDON-GFP

d. Prey: B_pDON-RFP

4 pDEST vectors in Cter:

a. Bait: MBD (SEQ ID N° 14)-GFP-A_pDON

b. Bait: MBD (SEQ ID N° 14)-RFP-A_pDON

c. Prey: GFP-B_pDON

d. Prey: RFP-B_pDON

For this system, the following 8 plasmids are obtained:

For the bait:

a. A-GFP- MBD (SEQ ID N° 14)

b. A-RFP- MBD (SEQ ID N° 14)

c. MBD (SEQ ID N° 14)-GFP-A

d. MBD (SEQ ID N° 14)-RFP-A

For the prey:

a. B-GFP

b. B-RFP

c. GFP-B

d. RFP-B

For a PPI A & B system, 16 plasmids can be generated in an exhaustive manner to characterize the mode of interaction of protein pairs for 2 given fluorophores: GFP and RFP.

In this case, the combinations are linked to the following factors

a. 2 proteins (A/B)

b. 2 types of combination (bait/prey)

c. 2 types of fusion (Nter/Cter)

d. 2 fluorophores (GFP/RFP)

The advantages of replicate steps a. and b. before step c. to determine the interactions between said one or more bait and said one or more candidate prey in the eukaryotic cell, and choose the right combination to be evaluate in the following steps to be closer to what happens in vivo, are, in the non-limited case of PPI interactions:

a. Improved cell microscopy results to identify and characterize the binding mode of the interaction (PPI (bait/prey) or RPI: bait only);

b. Choice of the best system to perform molecule screening to identify modulations (stabilization or disruption of the bait/prey interaction);

c. Possibility to generate more vectors for each of the considered fluorophores;

d. Reduction of the number of validation steps in classical molecular biology (e.g. restriction enzymes) and therefore increase in robustness and acceleration of obtaining the plasmids of interest;

e. Application of this approach to identify protein partners in the context of Target Identification.

This step of replication is relevant for any combination of bait and prey according to the invention (DNA, RNA or protein).

**General protocol for High-throughput analysis and evaluation system**

In the case of a protein bait, the cDNA sequences encoding for a known protein or a polypeptide chain called "bait" are inserted in a plasmid which directs, within the cell the synthesis of this bait fused to a microtubule binding protein (tau, MAP2) or domain linked to a projection domain to favor bait's accessibility to prey. The bait can additionally be fused to fluorescent label like a fluorescent protein (GFP, RFP, ...).

cDNA sequences encoding the "prey", a polypeptide or protein, are inserted in a plasmid to direct its expression within cell. The prey is possibly fused to a fluorescent label such as a fluorescent protein (i.e. GFP or XFP) for its detection in living cells via fluorescence microscopy.

Array of cell samples in 6 to 96-well plates are co-transfected with different preys (see figure 1) to identify various interacting partners for given bait at high throughput. An automatic detection system allows the recognition of the preys bound to microtubules so in order to obtain a Boolean output (interacting or not interacting).

4) Detection:

- i) For detection in living or fixed cells (figures 2 and 3), the co-expression of fluorescent bait and prey allows the detection by fluorescent videomicroscopy of the prey along dynamical microtubules, when interacting with the bait. Time lapse imaging for less than 1 minute (15 images per minute) is sufficient to detect microtubule structures with a high sensitivity. The sensitivity in living cells is enhanced compared to fixed cells since the highly dynamical microtubules can allow the detection of preys bound to the baits via differential fluorescence images of the prey (image at time t0 minus images at time t0+Œît, (Desforges et al.; An intercellular polyamine transfer via gap junctions regulates proliferation and response to stress in epithelial cells. Mol Biol Cell 24, 1529-1543; 2013), a classical technique to highlight microtubule movements which is useful here to withdraw the fluorescence background of non-interacting preys.

To better improve the accessibility of bait to prey and the sensitivity of detection, one can depolymerize microtubules in living cells, using reversible drugs like nocodazole, which allows the bait to explore freely the cytoplasm and to interact with the bait away from the microtubule surface. Then, once the microtubule-depolymerizing drug is washed out, microtubules rapidly regrow from the centrosome (<2 min), such a pattern being captured by videomicroscopy using the prey's fluorescence whenever an interaction between the bait and prey takes place. Confocal fluorescence microscopy or Total internal fluorescence microscopy (TIRF) can be used to enhance the sensitivity by reducing the fluorescence signal to noise ratio.

- ii) For detection in fixed cells (figure 3), the method allows the detection by fluorescence of native preys (endogenous proteins, endogenous RNA, endogenous DNA or other biomolecules) brought to microtubules thanks to their interaction with the bait. Detection can be done using classical optical fluorescence microscopy after labeling of the prey with specific antibodies (proteins chosen for being potentially interacting with the bait and using negative controls) or after in situ hybridization when the aim is to detect interaction of nucleic acids with the bait (RNA in particular including miRNA and mRNA). This option is also relevant when prey's fluorescent label (GFP, RFP or other labels) may hinder the prey-bait interactions or when prey over-expression may bias the results.

For detection in fixed cells co-expressing bait and fluorescent preys, fluorescent detection of preys brought to microtubules due to their interaction with the bait can be performed directly using classical optical microscope due to its fluorescent label.

In any case, microtubules appear as bright lines on fluorescence images, which renders the binding of the preys on them easily detectable by probing the appearance of $\mu$m-long bright lines using the prey's fluorescence signal.

Stable cell lines expressing the bait can also be used to improve the efficiency of co-expression and to facilitate the investigations of the interaction of a given protein bait. If the bait expression is toxic, an inducible expression system can be proposed in a stably transfected cell line to obtain at a given time a significant expression.

After step c. of contacting the said eukaryotic cell with the said compound, step d. is processed as discussed in this section for steps a. and b., and the results are compared in step e., to conclude in step f. whether the compound disrupt or stabilize said interaction.

## Tested compounds (step c.) and potential candidate for treating diseases

The in vitro method according to the invention comprises a step c. of contacting the said eukaryotic cell with the said compound, which eventually leads to modification of the interaction between the bait and the prey, and allow to conclude whether the effect of the said compound is the disruption or the stabilization of the interaction between said one or more bait and one or more candidate prey (step f.).

The contact of said eukaryotic cell with the said compound is made preferably by automated robot using 96-, 384-, 1536-well plates. The said eukaryotic cells are treated with various said compounds diluted in a solvent, such as DMSO. The duration of the treatment can range from a few minutes to several hours, or even days.

The contact of said eukaryotic cell with the said compound is made preferably by diluting the said compound in a solvent, more preferably a DMSO solvent. Said compound is diluted in different concentrations, depending of the tested compound.

The tested compound is then contacted with the said eukaryotic cell.

The duration of the contact between the tested compound and the said eukaryotic cell is made for several minutes to several days.

According to an embodiment of the invention, the duration of the contact between the tested compound and the said eukaryotic cell is chosen between : 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, or 59 minutes.

According to an embodiment of the invention, the duration of the contact between the tested compound and the said eukaryotic cell is chosen between: 1, 2, 3, 4, 5, 6, 7, 8, 9, 10, 11, 12, 13, 14, 15, 16, 17, 18, 19, 20, 21, 22, 23, or 24 hours.

According to an embodiment of the invention, the duration of the contact between the tested compound and the said eukaryotic cell is chosen between: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days.

Said compound may be a natural or chemical substance. In an embodiment, said compound is a small molecule.

This compound may be a known substance or came from a library of substance, based on *in vivo, in vitro,* or *in silico* data.

In a particular embodiment of the invention, the method is repeated with different concentrations of the compound in order to determine the IC50 of the compound for the disruption or the stabilization of the tested combination of one or more bait and one or more candidate prey.

In a particular embodiment of the invention, the method according to the invention allows to screen libraries of molecules, in order to identify potential candidates, and perform an IC50 on these relevant candidates.

According to the embodiment of this invention, the compound may be a medicament candidate, in case of the said one or more bait and said one or more candidate prey are characteristics of an interaction occurring in a specific pathology.

The invention allows to evaluate the ability of this compound to disrupt or stabilize an interaction between one or more bait and one or more candidate prey in a eukaryotic cell. As the combination of prey(s) and bait(s) may be characteristics of an interaction (or an absence of interaction) occurring in a specific pathology, this method allows to identify medicament candidate which would be able to act on an interaction (or an absence of interaction) and improve the treatment of the said pathology.

In the examples according to the invention, which are not limitative to the scope of combinations covered by this application, the applicants have been able to identify compounds having an impact on RPI interactions (protein bait and RNA prey) and on PPI interactions (protein bait and protein prey), proving the efficacy and the potential of the method according to the invention.

According to one embodiment, the invention relates to a compound identified by the in vitro method according to the invention, for use as a medicament.

In a particular embodiment of the invention, the one or more bait, and one or more candidate prey are characteristic of a cancer.

In a preferred embodiment of the invention, the cancer is selected from the group comprising or consisting of : Colorectal cancer, Malignant brain tumors, Lung cancer, Drug resistance cancer, Pancreatic cancer.

In this embodiment, the method and uses according to the invention are able to conclude whether the effect of the tested compounds is the disruption or the stabilization of the interaction, and conclude therefore if one or more tested compounds may be of interest as potential medicament candidates in the type of cancer characteristics of the combination of bait(s) and prey(s).

According to one embodiment, the compound is for use in treating a cancer in a subject.

In a particular embodiment of the invention, the one or more bait, preferably a protein bait, and one or more candidate prey are characteristic of a proteinopathy.

According to one embodiment, the compound is for use in treating a proteinopathy in a subject.

In a preferred embodiment of the invention, the proteinopathy is selected from the group comprising or consisting of: Alzheimer's disease, Parkinson's disease, Huntington's disease, amyotrophic lateral sclerosis, frontotemporal lobe dementia, TDP-43 or FUS proteinopathies.

In a more preferred embodiment of the invention, the proteinopathy is a TDP-43 proteinopathy, preferably the proteinopathy is amyotrophic lateral sclerosis (ALS) or frontotemporal lobar degeneration (FTLD), and wherein the protein of interest is TDP-43.

According to one embodiment, the mutated form of TDP-43 is selected from the group comprising or consisting of: A90V, D169G, N267S, G287S, G290A, S292N, G294A/V, G295C/R/S, G298S, M311V, A315T/E, A321G/V, Q331K, S332N, G335D, M337V, Q343R, N345K, G348C/R/V, N352S/T, G357R/S, R361S/T, P363A, G368S, Y347X, G376D, N378D/S, S379C/P, A382P/T, I383V, G384R, W385G, N390S/D and S393L.

The method according to the invention has been able to identify compounds related to TDP-43 proteinopathies.

A number of mutations in the C-terminus domain of TDP-43 favors the transition from a liquid state of TDP-43, wherein the protein is present in reversible liquid subcellular compartments, to a solid aggregation state, wherein the protein accumulates to form solid and irreversible aggregates in the cell.

The process according to the WO2016012451 patent family has been used to investigate the mutations involved in the transition to solid aggregate state of TDP-43, which is characteristic of ALS pathology.

Since the mutated forms of TDP-43 showed various defects in the cellular tests, the approach was to find compounds capable of restoring these defects.

134 compounds from a library of inhibitors of kinase or of phosphatase have been tested with the method according to the invention, with the system TDP43-GFP-MBD (SEQ ID N° 14) [wt]/SE3-RFP-MBD (SEQ ID N° 14).

After a first screening, 13 compounds that either increased the demixing of the proteins (i.e. indicative of the solid aggregation state of the mutated form of the TDP-43), or on the reverse, increased the mixing of the proteins (i.e. indicative of the liquid state of the mutated form of the TDP-43) have been selected, having no toxicity problem.

The 13 selected compounds have then been tested in the three cellular tests, i.e. enrichment of wt TDP-43 in stress granules and their subcellular localization in presence of the compounds to be tested, and the effects of the compounds to be tested on the alternative splicing of the cystic fibrosis transmembrane conductance regulator (CFTR) gene by wt TDP-43. Finally, the effects of the 13 compounds on the aggregation of the G146A mutant of TDP-43 has been tested.

The inhibitors of kinase increased the demixing of the proteins and the aggregation of the mutant G146A while the inhibitors of phosphatase increased the mixing of the proteins and decreased the aggregation of the mutant G146A. Among these compounds, 4 compounds showed an effect on at least two of the four tests: 3 inhibitors of kinase (Ro-mesylate, PKRi, 5-iodotubercidin) and one inhibitor of phosphatase (9,10-Phenanthrenequinone). Therefore, these results show that the inhibitors of phosphatase identified by this method are able to restore defects observed in ALS, such as the accumulation of mutated forms of TDP-43.

These results show that the method according to the invention could be used to test mutated forms of proteins of interest, in particular proteins that accumulate in subjects suffering from proteinopathies, and to identify compounds that could be used for treating said proteinopathies.

## Examples

### EXAMPLE 1: Targeting Protein:Protein Interactions to identify modulators in a cellular environment

We chose the widely studied system p53/MDM2 (Moll and Petrenko, 2003; Nayak et al., 2018; Zhao et al., 2015) as a proof of concept to investigate the performance of the MT bench technology to screen PPI inhibitors using High-Content Screening systems. High-Content Screening (HCS) is an image-based method used in drug discovery to identify molecules (biologics or small molecules) that alter a phenotype using simultaneously several parameters of the cell as a readout (Fraietta and Gasparri, 2016; Mattiazzi Usaj et al., 2016). An analysis pipeline has been developed to measure the co-localization on individual cells from HCS images by extracting a correlation score. The correlation score has been calculated using the Pearson Correlation Coefficient (PCC) between the enhanced texture intensities from the prey and the bait. We measured a good Z'-factor of 0.69 (between 0.5 and 1, the assay is considered excellent) (Zhang, 1999; Zhang et al., 2000), reflecting the robustness of the technology to screen small inhibitors of p53/MDM2 with high content capacity. We then validated that the interaction detected on microtubule relies on specific residues of the MDM2-binding site with p53. Having demonstrated the validity of the MT bench to quantify p53/MDM2 interaction, we started to screen a small set of 10 molecules with known activity on MDM2 as previously characterized by HTRF assay (Gicquel et al., 2018) for hit identification. Both false negative and false positive molecules identified from HTRF assay were successfully detected and were also confirmed independently by measuring the accumulation of P53 and P53-related proteins. The MT bench assay was also used to calculate IC50 from HCS images. We obtained a cellular IC50 of 74nM and 426nM for the known p53/MDM2 inhibitors, RG7112 and Nutlin-3a (Vu et al., 2013). These values are consistent with those found in the literature from in vitro HTRF assay (Liu et al., 2019; Neochoritis et al., 2019a;

Vu et al., 2013) but also from the cellular nanoBRET assay (Itatani and Olsen). We also used the results to characterize the inhibition constant for all the molecules tested that allow to do a cellular structural activity relationship (SAR). Altogether, the results presented in this study validate the MT bench technology in HCS setting as a robust method to screen and characterize PPI modulators in cells.

### METHODS AND MATERIALS:

#### Generation of genetic constructs

The cloning of gene of interest was made using the gateway system from Invitrogen. A set of pDEST plasmid, derived from pDEST-CMV-N-EGFP and pDEST-CMV-C-EGFP has been designed, using synthetic genes purchased from Twist bioscience, in order to produce a combination of constructs with N-terminus and C-terminus fusion proteins for the prey (protein of interest fused with eGFP protein) and the bait (protein of interest fused with RFP fluorophore and a microtubule binding domain (MBD (SEQ ID N° 14)) corresponding to the longest isoform of the human Tau protein as previously described(Boca et al., 2015)). Human p53 and MDM2 full length cDNA were amplified by PCR with primers containing attB1 and attB2 sites and either a kozak sequence and without a stop codon to generate a "fusion" entry clone in pDONR221 for a recombination into a pDEST-CMV-C vector (for a C-terminus fusion protein) or without the ATG but with a stop codon to generate a "close" entry clone for a recombination into a pDEST-CMV-N vector (for a N-terminus fusion protein).

The construct of p53 (SEQ ID N°15) as the bait with a C-terminus fusion of RFP-MBD (p53-FL_C_RFP- MBD) and the construct of MDM2 (SEQ ID N°21) as the prey with a C-terminus fusion of GFP (MDM2- FL_C_GFP) were selected for the MT bench. The p53 mutants were obtained via subcloning from synthesized genes purchased from Twist Bioscience to generate the mutant constructs: p53F19A (SEQ ID N°17) C_RFP-MBD, p53W23A (SEQ ID N°16)_C_RFP-MBD with single mutation; p53F19AW23A (SEQ ID N°18)_C_RFP-MBD, p53W23AL26A(SEQ ID N°19)_C_RFP-MBD with double mutations and p53F19AW23AL26A(SEQ ID N°20)_C_RFP-MBD with triple mutations. All the plasmids generated were purified by NucleoSpin Plasmid DNA Purification kit from MACHEREY- NAGEL and the integrity of the different DNA sequences was verified by sequencing from Eurofins Genomics.

Generation of cellular plate

The MT bench was done using Human Osteosarcoma (U-2 OS) cell line which is the reference for cellular imagery. U-2 OS cells were cultured at 37°C in a humidified atmosphere with 5% CO2 in Dulbecco's modified Eagle's medium (DMEM, Life Technologies) supplemented with 10% FBS (Fetal Bovine Serum, Life Technologies) and 1% penicillin/streptomycin.

The generation of the cellular plate (seeding, transfection, treatment) was done automatically using the liquid handler BRAVO from Agilent equipped with a 96-LT (Large Tips) head. U-2 OS cells were seeded on black 96-well plates cell carrier ultra (PerkinElmer) at a density of 18.000 cells per well and after 24h incubation in a humidified incubator at 37°C with 5% CO2 were co-transfected with 12μg human p53-FL(SEQ ID N°15)_C_RFP- MBD and 03μg human MDM2-FL(SEQ ID N°21)_C_GFP plasmids for the positive control (untreated condition) or 12μg human p53-F19AW23AL26A(SEQ ID N°20)_C_RFP-MBD and 0.3μg human MDM2-FL(SEQ ID N°21)_C_GFP plasmids corresponding to the negative control (non-interacting condition / treated condition) using 0.5μg Lipofectamine 2000 (Invitrogen) in optiMEM for 18h.

For hit identification, cells were treated using 0.1% DMSO for the control wells and 2 concentrations (1μM and 10μM) of RG7112, Nutlin-3a and 8 other compounds (Rac-7b", Rac-4a", Rac-4c', Rac-4d', Rac-8', (-)-8', (+)-8', Rac-MG-1133) diluted from a 50mM or 10mM stock solution in culture medium during 2h at 37°C. For $IC_{50}$, cell treatments were performed by adding 0.1% DMSO for the control wells and with 10 concentrations ranging from 0.001μM to 25μM or 0.01μM to 100μM of the same compounds to the culture medium during 2h at 37°C.

After 18h of transfection, cells were fixed with ice-cold methanol 100% for 10min at -20°C, washed with PBS and then further fixed with 4% Para FormAldehyde (PFA) in PBS freshly prepared for 10min at RT. This double fixation (Methanol/PFA) is used as the best method to reveal microtubules and co-localization events. After washing with PBS, cell nuclei were stained with DAPI (0.1μg/mL) for 5min at RT and samples kept into PBS for fluorescence microscopy imaging. The cellular fluorescent signal was acquired on HCS imaging system Opera Phenix from PerkinElmer, using a 40X water immersion 1.1NA objective and with Harmony® software.

Image and statistical analysis

To quantify the co-localization level between a protein bait fused with MBD and a putative protein prey, we developed an analysis pipeline, adapted from a method previously described (French et al., 2008). After image acquisition, successive building blocks from Harmony® software was used for image segmentation (find nuclei, find cytoplasm, and select population through threshold on GFP and RFP maximum and mean intensities, as well as cell area). Both images were then filtered using SER Ridge filters to highlight microtubule structures in the cytoplasm.

To quantify the degree of co-localization between fluorophores we used the Pearson correlation coefficient (PCC)(Benesty et al., 2009). We have also used the Mander's overlap coefficient (MOC)(Manders et al., 1993) (data not shown). While, these two coefficients are mathematical similar, the PCC seems to be more precise to quantify the co-localization by using the deviation from the mean (Adler and Parmryd, 2010). The PCC is a well-established correlation score to measure co-localization and calculated as follows

$$ r = \frac{\sum (R_i - R_{av}) \cdot (G_i - G_{av})}{\sqrt{\sum (R_i - R_{av})^2 \cdot \sum (G_i - G_{av})^2}} $$

where $R_i$ and $G_i$ are the pixel intensity values and $R_{av}$ and $G_{av}$ are the means of red and green channel, respectively. The values are range between +1 (perfect correlation) to -1 (perfect inverse correlation), with 0 being the absence of co-localization. Thus, the PCC values reflect the co-localization between bait and prey proteins into cytoplasm, translating the interaction on the microtubule network.

In the case of hit identification, the raw data (PCC scores) were normalized to allow a comparison between experimental plates (Kevorkov and Makarenkov, 2005). This normalization was done by extrapolating the PCC values relatively to the controls, by using the mean of negative control wells and the mean of positive control wells.

The Z'-factor is related to the separation band between the distribution of positive and negative controls values. It is calculated with the following formula:

$$Z' = 1 - \frac{3\sigma C^{+} + 3\sigma C^{-}}{|\mu C^{+} - \mu C^{-}|}$$

where $\sigma C+$ and $\sigma C-$ are the standard deviations of the positive and negative controls, respectively; $\mu C+$ and $\mu C-$ their averages (Zhang, 1999; Zhang et al., 2000). The Z-factor is the equivalent of the Z' factor when sample is used instead of negative control. The quality of an HTS assay is categorized according to the value of the Z' and Z-factor obtained. The value of 0.5 is the commonly accepted threshold required for a good quality screening.

The means ± SD of an independent experiment in quadruplicate were represented as histograms. R statistical software and GraphPad Prism® software were used for computing data, statistical analysis, and plotting. Statistical comparisons were performed with Student t-test on GraphPad Prism®. The followed symbols were used: * $p < 0.1$, ** $p < 0.01$, *** $p < 0.001$, **** $p < 0.0001$.

Western blotting

Human Osteosarcoma (U-2 OS) were cultured in 6-well plates at a confluence of $0.5 \times 10^6$ cells/well in DMEM (Life Technologies) supplemented with 10% FBS (Life Technologies) and 1% penicillin/streptomycin under humidified atmosphere at 37°C and with 5% CO2. After 24h culture, compounds were added at the final concentrations of 10μM). After 6h of treatment, cells were lysed with RIPA buffer (Sigma) supplemented with protease inhibitor cocktail (Sigma). After protein dosage by using BCA Protein Assay Kit (Pierce™), equal amount of protein lysate (10μg) was loaded after denaturation into the well of a 12% SDS- PAGE gels. After protein separation by electrophoresis (120V, 45min), proteins were transferred to PVDF membrane (Amersham Hybond-P Membrane, GE Heathcare) by electroblotting (100V, 1h15) using Trans-Blot®Turbo™ Blotting System. The membranes were blocked with 5% skim milk in 0.1% Tween-PBS (T-PBS) for 2h at room temperature. Afterwards, membranes were probed with the following primary antibodies: mouse monoclonal anti-human p53 (DO-1, #sc-126, Santa Cruz Biotechnology Inc), rabbit anti- human p21 (C-19, #sc-397, Santa Cruz Biotechnology Inc) and mouse monoclonal anti- actin (#A5441, Sigma). As secondary antibodies, we used the horseradish peroxidase (HRP) conjugated goat anti-mouse IgG (#PO447, Dako) and goat anti-rabbit IgG (#AP187P, Millipore). The blots were reveled using ECL Western Blotting Kit (Bio-Rad), visualized and quantified using ChemiDoc MP imaging System (Bio-Rad).

**RESULTS:**

High-Content Screening of p53/MDM2 interaction with the microtubule bench cell- based assay

To implement the MT bench technology (Figure 4A) on HCS system, we used a well-known PPI, p53-MDM2(Moll and Petrenko, 2003; Nayak et al., 2018; Zhao et al., 2015) as a model. To analyze the interaction between p53 and MDM2, we have transiently expressed both p53 and MDM2 fusion proteins in U-2OS cells. We have been able to translate the assay from microscope to HCS system allowing a robust quantification of the binding events.

The bait protein, p53 full-length (SEQ ID N°15) is fused to both RFP (Red Fluorescent Protein) and MBD (Microtubule Binding Domain) (SEQ ID N°14) from a MAP (Microtubule Associated Protein) to bring P53 on microtubule and allow the observation of the bait along the microtubule network in cells using the red channel. In contrast, the prey protein, MDM2 is not brought on microtubule and solely fused to GFP (Green Fluorescent Protein) to enable its detection by fluorescence microscopy with the green channel. When the red and green channels are merged, both fluorescent signals co-localized along the same microtubules, which correspond to an interaction between the bait, p53, and the prey, MDM2 (Figure 4A). Although, the natural sub-localization of p53 and MDM2 is mostly the nucleus, the ability of the technology to relocate nuclear proteins in the cytoplasm made it possible to detect p53/MDM2 interaction along microtubules. As a negative control, the plasmid containing the full length p53 as the bait (p53-FL(SEQ ID N°15)_C_RFP-MBD) was mutated at the three hotspot residues (Phe19, Trp23 and Leu26) that abrogate the interaction with MDM2 (Bista et al., 2013). We then observed a loss of co-localization at the microtubules with a re- localization of MDM2 into the nucleus (Figure 4B). Interestingly, a single mutation of one of this three hotspot residue is sufficient to abrogate more than 90% of the interaction detected on microtubules (Figure 5).

To provide an interaction score, we developed an image analysis pipeline to measure the co-localization of p53 and MDM2 on microtubules with the Harmony® software from PerkinElmer, as described in the material and method section (Figure 6). To this end, we measured the Pearson correlation coefficient (PCC)(Adler and Parmryd, 2010; Benesty et al., 2009) in the cytoplasm of each co-transfected cell, and then computed the average PCC per well giving a statistically reliable score (Figure 6). After having detected the interaction between p53 and MDM2 in a cellular context, we used a well characterized MDM2 inhibitor, RG7112 (Liu et al., 2019; Vu et al., 2013) to probe whether perturbations by small molecules could be quantified with the MT bench. When cells were treated with increasing concentrations of RG7112 ([0.05μM], [0.5μM], [5μM] and [50μM]), we observed a decrease in the p53 and MDM2 interaction score with the MT bench assay in a dose-dependent manner (Figure 4C). The dose-dependent response of RG7112 is characterized by a weak inhibition of the interaction at low concentration ([0.05μM]) and a strong inhibition of the interaction at high concentration ([5μM] and [50μM]), passing through moderate inhibition of the interaction at medium concentration ([0.5μM]) (Figure 4C). In addition, we noticed in the green channel the gradual disappearance of MDM2 on the microtubule network reflecting the progressive loss of interaction between p53 and MDM2, induced by the small molecule. Interestingly, with the MT bench assay, the presence of inhibitors of p53/MDM2 triggers not only the loss of interaction but also correlates with a progressive re-localization of MDM2 into the nucleus.

We then tested whether the MT bench assay could be used for screening PPI inhibitors with HCS. We then measured the widely used Z'-factor on a 96 well plate format, a statistical parameter to validate the suitability of an assay for high throughput screening (Zhang, 1999) (see Methods). When using the p53 triple mutant (p53- F19AW23AL26A) as a negative control, the resulting Z'-factor is 0.69 which is above 0.5, the threshold for assay considered excellent (Figure 15A). The system has been also implemented on 384 well plate format with a Z' = 0.65 (Figure 7A). It is worthy to note that the Z'-factor calculated after using G3BP1 protein, used as negative control bait, is 0.59. This value is in the same range than with p53 triple mutant that abrogate the PPI meaning that, without prior knowledge about hot spot residues preventing specific PPI, we could use another non-related protein as negative control (Figure 7B-C). In addition, we measured the Z-factor (Zhang et al., 2000) when the negative control corresponds to a sample in which a full disruption of an interacting complex by a characterized inhibitor has been obtained. Here we measured a minimum co-localization score with 5 μM of RG7112 and measured a Z'-factor of 0.67 when this condition was used as negative control (Figure 15B). Together, this set of data proved that set up of the system p53/MDM2, using the MT bench technology on HCS, is robust, reproducible, and sensitive for hit identification.

## DISCUSSION:

The direct visualization and quantification of full-length protein interactions in human cells offer certain advantages compare to other methods. Notably, we show that the MT bench technology allows the identification of hotspot residues and small molecule modulators of PPI, which represent strategically important targets for drug discovery. Moreover, we are also able to measure dose-response effect and extract their cellular activities (IC$_{50}$). Such cellular Structure Activity Relationship (SAR) information could pave the way for the optimization of modulators, by using chemoinformatic tools.

An important feature of the MT bench technology is that it could reduce the number of false hits compared to HTRF probably due to its high sensitivity and the fact the interaction involved full length proteins and occurs in human cells. Indeed, based on the screen reported here, the molecule Rac-MG-1133 was not identified as a potential p53/MDM2 disruptor in HTRF but significantly inhibit p53/MDM2 interactions in cells. Rac-MG-1133 can thus be considered as a false negative. The MT bench assay can discriminate the effect of enantiomers from the racemic mixture as exemplified for the molecule Rac-8. The dextrorotary enantiomer (+)-8' induced a strong inhibition on both assays whereas the levorotary enantiomer (-)-8' is a false positive in the MT bench assay most likely because this enantiomer is not efficient in cells while it still displays an inhibition on HTRF *in vitro.*

The robustness of the MT bench technology lies on the fact that it based on single cell analysis. Therefore, the output per well is the average of hundreds of events, which results in quantitatively accurate scoring of the interactions between two proteins and thus its perturbation by a modulator. Indeed, the similar Z'-factors using either a non-binding protein as a bait, a non-interacting mutant as a control or a small molecule resulting to a complete disruption of the interaction shows the reproducibility of the MT bench assay (Figure 15A-B and Figure 7C).

The simplicity and flexibility of the technology working with full length protein in cells, in addition to the variety of negative controls that can be used, makes it possible to screen protein interactions without prior structural data or knowledge on the interaction interfaces. Another advantage of using full length protein lies on the fact that either orthosteric or allosteric modulators can be identified. The MT bench can also be used to confirm the interaction between two partners and to perform hot spot identification in cell which provide valuable information for the discovery of PPI inhibitors using virtual screening approaches (Guo et al., 2014).

Here, we showed that The MT bench is a reliable cell-based technology for the discovery of PPI inhibitors, and it could provide a solution for the discovery of new chemical scaffolds (Neochoritis et al., 2019b) with improved ADMET properties and help for clinical development of MDM2/MDMX inhibitors for cancer treatment.

To conclude, we have shown that the MT bench is a robust, quantitative and sensitive cell-based screening assay. This technology combined with *in silico* expertise could greatly ease early drug discovery, in time efficient manner, for challenging targets such as PPIs. By reducing the risk of false positives and false negatives at the hit identification phase and the characterization of the mechanism of action of PPI modulators, the MT bench can facilitate the lead optimization of small molecules with properties suitable for pre-clinical studies.

**EXAMPLE 2: Targeting Protein:Protein Interactions to identify modulators and determinine IC50 data in cells.**

**METHODS AND MATERIALS:**

Generation of genetic constructs

The cloning of genes of interest was made using the gateway system from Invitrogen. A set of pDEST plasmid, derived from pDEST-CMV-N-EGFP and pDEST-CMV-C-EGFP has been designed, using synthetic genes purchased from Twist bioscience, in order to produce a combination of constructs with N-terminus and C-terminus fusion proteins for the prey (protein of interest fused with eGFP protein) and the bait (protein of interest fused with RFP fluorophore and a microtubule binding domain (MBD)(SEQ ID N°14) corresponding to the longest isoform of the human Tau protein as previously described(Boca et al., 2015)). Human p53 and MDM2 full length (SEQ ID N°21) cDNA were amplified by PCR with primers containing attB1 and attB2 sites and either a kozak sequence and without a stop codon to generate a "fusion" entry clone in pDONR221 for a recombination into a pDEST-CMV-C vector (for a C-terminus fusion protein) or without the ATG but with a stop codon to generate a "close" entry clone for a recombination into a pDEST-CMV-N vector (for a N-terminus fusion protein).

The construct of p53 as the bait with a C-terminus fusion of RFP-MBD (p53-FL(SEQ ID N°15)-RFP-MBD(SEQ ID N°14)) and the construct of MDM2(SEQ ID N°21) as the prey with a C-terminus fusion of GFP (MDM2-FL-GFP) were selected for the MT bench. The p53 mutants were obtained via subcloning from synthesized genes purchased from Twist Bioscience to generate the mutant constructs: p53F19A-RFP-MBD, p53W23A-RFP-MBD with single mutation; p53F19AW23A-RFP-MBD, p53W23AL26A-RFP-MBD with double mutations and p53F19AW23AL26A-RFP-MBD with triple mutations. All the plasmids generated were purified by NucleoSpin Plasmid DNA Purification kit from MACHEREY-NAGEL and the integrity of the different DNA sequences was verified by sequencing from Eurofins Genomics.

Generation of cellular plate

The MT bench was done using Human Osteosarcoma (U-2 OS) cell line. U-2 OS cells were cultured at 37°C in a humidified atmosphere with 5% CO2 in Dulbecco's modified Eagle's medium (DMEM, Life Technologies) supplemented with 10% FBS (Fetal Bovine Serum, Life Technologies) and 1% penicillin/streptomycin.

The generation of the cellular plate (seeding, transfection, treatment) was done automatically using the liquid handler BRAVO from Agilent equipped with a 96-LT (Large Tips) head. U-2 OS cells were seeded on black 96-well plates cell carrier ultra (PerkinElmer) at a density of 18.000 cells per well and after 24h incubation in a humidified incubator at 37°C with 5% CO2 were co-transfected with the indicated quantities of plasmids of interest using 0.5μg Lipofectamine 2000 (Invitrogen) in optiMEM for 18h.

For IC50, cell treatments were performed by adding 0.1% DMSO for the control wells and with 10 concentrations ranging from 0.001μM to 25μM of RG7112 to the culture medium during 2h at 37°C.

After 18h of transfection, cells were fixed with ice-cold methanol 100% for 10min at -20°C, washed with PBS and then further fixed with 4% Para FormAldehyde (PFA) in PBS freshly prepared for 10min at RT. This double fixation (Methanol/PFA) is used as the best method to reveal microtubules and co-localization events. After washing with PBS, cell nuclei were stained with DAPI (0.1μg/mL) for 5min at RT and samples kept into PBS for fluorescence microscopy imaging. The cellular fluorescent signal was acquired on HCS imaging system Opera Phenix from PerkinElmer, using a 40X water immersion 1.1NA objective and with Harmony® software.

Image and statistical analysis

To quantify the enrichment level of a protein prey on a protein bait fused with MBD, we developed an analysis pipeline, adapted from a method previously described (French et al., 2008). Image Acquisitions and statistical analysis of the cellular fluorescent signals were acquired on the high-content imaging system Opera Phenix Plus from Perkin Elmer on 40x water immersion objectives with a numerical aperture of 1.1, allowing us to obtain a good resolution in the confocal mode. Several fields of views were taken for each well resulting in thousands of cells to be analyzed, by well, in a 96 or 384 wells plate format to have the strongest statistical significance. The data were extracted with the SImA® software using an analysis pipeline containing successive building blocks for image segmentation, selection of population of interest, and calculation of signal enrichment on the microtubules (MTs). The enrichment is calculated on identified spots using the RFP (Red Fluorescence Protein) channel that corresponds to the signal of the bait protein forced to be localized at MT due to its fusion with a microtubule binding domain (MBD). Spots representing segments of MTs were selected based on their shape and on the intensity of the RFP channel signals (corresponding to the presence of the bait on MT). The other protein was detected with GFP (Green Fluorescence Protein) and could be brought on MT due to their potential interaction with the bait. The calculated RFP and GFP intensities on the spots and in the cytoplasm were extracted from the SImA® software and treated subsequently to measure the slope of the prey protein enrichment on MTs (mean spot intensity divided by mean cytoplasm intensity versus mean bait spot intensity).

Spotfire® data analysis software was used for computing data, statistical analysis, and plotting. Raw data were normalized using a min-max normalization (with positive and negative controls as max and min) to allow a comparison between experimental plates (Kevorkov and Makarenkov, 2005).

## RESULTS AND ANALYSIS:

To implement the MT bench technology on HCS system, we used a well-known PPI, p53-MDM2(Moll and Petrenko, 2003; Nayak et al., 2018; Zhao et al., 2015) as a model. To analyze the interaction between p53 and MDM2, we have transiently expressed both p53 and MDM2 fusion proteins in U-2OS cells.

The bait protein, p53 full-length is fused to both RFP (Red Fluorescent Protein) and MBD (Microtubule Binding Domain) from a MAP (Microtubule Associated Protein) to bring P53 on microtubule and allow the observation of the bait along the microtubule network in cells using the red channel. In contrast, the prey protein, MDM2 is not brought on microtubule and solely fused to GFP (Green Fluorescent Protein) to enable its detection by fluorescence microscopy with the green channel (Figure 4A).

When the red and green channels are merged, both fluorescent signals co-localized along the same microtubules, which corresponds to an interaction between the bait, p53, and the prey, MDM2. Although, the natural sub-localization of p53 and MDM2 is mostly the nucleus, the ability of the technology to relocate nuclear proteins in the cytoplasm made it possible to detect p53/MDM2 interaction along microtubules. The plasmid containing the full length p53 as the bait (p53-FL-RFP-MBD) was mutated When the red and green channels are merged, both fluorescent signals co-localized along the same microtubules, which corresponds to an interaction between the bait, p53, and the prey, MDM2. Although, the natural sub-localization of p53 and MDM2 is mostly the nucleus, the ability of the technology to relocate nuclear proteins in the cytoplasm made it possible to detect p53/MDM2 interaction along microtubules. The plasmid containing the full length p53 as the bait (p53-FL-RFP-MBD) was mutated at the three hotspot residues (Phe19, Trp23 and Leu26) that abrogate the interaction with MDM2 (Bista et al., 2013). We then observed a loss of co-localization at the microtubules with a re-localization of MDM2 into the nucleus (Figure 4B).

In order to quantitatively estimate the protein-protein interaction, we developed an image analysis pipeline to measure the enrichment of MDM2 on microtubules via its interaction with p53, as described in the material and method section and we tested whether the MT bench assay could be used for screening PPI inhibitors with HCS.

We first measured the enrichment to the microtubules of MDM2 when overexpressing different p53 mutants (Figure 8).

We assessed the enrichment level in each condition (Figure 9). The expression of p53 mutants led to a lesser enrichment on microtubules, closer to the negative control enrichment level. In conditions where the single mutant p53-L26A-RFP-MBD or the triple mutant p53-F19AW32AL26A-RFP-MBD were expressed, MDM2 was never found at the microtubules meaning that these mutants totally abrogate the protein-protein interaction. These data show the that the MT bench technology allows the identification of hotspot residues modulators of PPI.

After having detected the interaction between p53 and MDM2, we used a well characterized MDM2 inhibitor, RG7112 (Liu et al., 2019; Vu et al., 2013) to probe whether perturbations by small molecules could be quantified with the MT bench. We determined the IC50 of this molecule in cells over 10 concentrations using the MT bench assay. The IC50 value calculated for RG7112, is $0.074 \pm 0.033\mu M$, which is consistent with the IC50 reported from HTRF assays (IC50 = $0.018\mu M$) (Vu et al., 2013). Some cellular data have also been reported using MTT assay for RG7112 (IC50 = $0.4\mu M$) (Vu et al., 2013). (These results demonstrate that the MT bench technology is sensitive enough to detect perturbations in cells even at the lowest concentration of inhibitor, allowing us to get more accurate IC50 data in cells.

**EXAMPLE 3: Targeting RNA:Protein Interactions to identify modulators and determine IC50 data in cells.**

METHODS AND MATERIALS:

Generation of genetic constructs

The cloning of genes of interest was made using the gateway system from Invitrogen. A set of pDEST plasmids (pDEST-N-MBD-EGFP and pDEST-CMV-C-EGFP-MBD), derived from pDEST-CMV-N-EGFP and pDEST-CMV-C-EGFP has been designed, using synthetic genes purchased from Twist bioscience, in order to produce a combination of constructs where the bait protein is fused to EGFP in either the N-terminus or the C-terminus and then fused to a microtubule binding domain (MBD) corresponding to the longest isoform of the human Tau protein as previously described (Boca et al., 2015)). Human YB1FL (full length) cDNA was amplified by PCR with primers containing attB1 and attB2 sites and either a kozak sequence and without a stop codon to generate a "fusion" entry clone in pDONR221 for a recombination into a pDEST-C-GFP-MBD vector (for a C-terminus fusion protein) or without the ATG but with a stop codon to generate a "close" entry clone for a recombination into a pDEST-CMV-N vector (for a N-terminus fusion protein). YB1∆CSD (SEQ ID N°23) (where the amino acids 52-129 corresponding to the CSD domain of YB1 were deleted) cDNA was synthesized by Eurofins Genomics and the constructs were then produced in a similar way as YB1 FL(SEQ ID N°22).

All the plasmids generated were purified by NucleoSpin Plasmid DNA Purification kit from MACHEREY-NAGEL and the integrity of the different DNA sequences was verified by sequencing from Eurofins Genomics.

Generation of cellular plate

The MT bench was done using HeLa cells (ATCC CCL-2). HeLa cells were cultured at 37°C in a humidified atmosphere with 5% CO2 in Dulbecco's modified Eagle's medium (DMEM, Life Technologies) supplemented with 10% FBS (Fetal Bovine Serum, Life Technologies) and 1% penicillin/streptomycin.

The generation of the cellular plate (seeding, transfection, treatment) was done automatically using the liquid handler BRAVO from Agilent equipped with a 96-LT (Large Tips) head. HeLa cells were seeded on black 384-well plates cell carrier ultra (PerkinElmer) at a density of 5000 cells per well and after 24h incubation in a humidified incubator at 37°C with 5% CO2 were transfected with 0.5µg of plasmids of interest using 0.1µg Lipofectamine 2000 (Invitrogen) in optiMEM for 18h.

Cells were then first fixed with ice-cold methanol 100% for 10 min at -20 °C, washed with PBS and then further fixed with 4% PFA in PBS freshly prepared for 10 min at RT. After fixation, cells were incubated with oligo-dT-[Cy3], diluted in SSC 2X, 1 mg.ml-1 yeast tRNA, 0.005% BSA, 10% dextran sulfate, 25% formamide, for 2 h at 37 °C for RNA visualization. Wash steps were performed using 4X and then 2X SSC buffer (0.88% sodium citrate, 1.75% NaCl, pH 7.0). Cell nuclei were stained with DAPI (0.1 µM) for 5 min at RT.

After washing with PBS, cell nuclei were stained with DAPI (0.1µg/mL) for 5min at RT and samples kept into PBS for fluorescence microscopy imaging. The cellular fluorescent signal was acquired on HCS imaging system Opera Phenix Plus from PerkinElmer, using a 40X water immersion 1.1NA objective and with Harmony® software.

Image analysis

To quantify the enrichment level of a mRNA prey on a protein bait fused with MBD, we developed an analysis pipeline, adapted from a method previously described (French et al., 2008). Image Acquisitions and statistical analysis of the cellular fluorescent signals were acquired on the high-content imaging system Opera Phenix Plus from Perkin Elmer on 40x water immersion objectives with a numerical aperture of 1.1, allowing us to obtain a good resolution in the confocal mode. 20 fields of views were taken for each well resulting in thousands of cells to be analyzed, by well, in order to have the strongest statistical significance. The data were extracted with the SImA® software using an analysis pipeline containing successive building blocks for image segmentation, selection of population of interest, and calculation of signal enrichment on the microtubules (MTs). The enrichment is calculated on identified spots using the GFP (Green Fluorescent Protein) channel that corresponds to the signal of the bait protein forced to be localized at MT due to its fusion with a microtubule binding domain (MBD). Spots representing segments of MTs were selected based on their shape. mRNA were detected with Cy3-labelled poly(dT) and could be brought on MT due to their potential interaction with the bait. The calculated GFP and Cy3 intensities in the spots and in the cytoplasm were extracted from the SImA® software and treated subsequently in order to measure the relative enrichment of the mRNA on MTs (mean spot intensity divided by mean cytoplasm intensity (GFP) on mean bait spot intensity divided by mean cytoplasm intensity (Cy3)).

Spotfire® datas analysis software was used for computing data, statistical analysis, and plotting. Raw data were normalized using a min-max normalization (with positive and negative controls as max and min) to allow a comparison between experimental plates (Kevorkov and Makarenkov, 2005).

Statistical analysis

In order to determine whether there was a significant difference in relative enrichment between conditions, we conducted a Student's t-test. This statistical test allows us to compare the mean values of two groups and determine whether the difference between them is statistically significant. We formulated the following hypotheses for the t-test:

Null hypothesis (H0): There is no significant difference in relative enrichment between conditions.

Alternative hypothesis (H1): There is a significant difference in relative enrichment between conditions.

To ensure that the t-test is reliable, it is necessary to meet certain conditions. First, the observations within each group should be independent of each other. In our experiment, we used multiple replicates for each condition, which helps to ensure independence of the observations.

Next, the data should be normally distributed within each group. We checked the normality of the data using histograms and normal probability plots, and found that the data were approximately normally distributed.

We also checked for equal variances using the F-test and found that the variances of the two groups were not significantly different. This is important because the t-test assumes equal variances, and failure to meet this assumption can affect the reliability of the test.

Finally, we checked the sample sizes of the two groups and found that they were equal. This is important because the t-test assumes equal sample sizes, and failure to meet this assumption can also affect the reliability of the test.

Overall, we believe that the conditions for the t-test are satisfied in our experiments, and that the results of the test can be trusted to accurately compare the relative enrichment of the two conditions.

Based on the results of the t-test, we can conclude that the null hypothesis should be rejected. The p-value for the test was less than 0.001, which indicates that the difference in relative enrichment between the two experiments is statistically significant. This suggests that there is a real difference between the two groups, and not just random variation.

We use a geometric mean with the negative control as a reference for normalization to compare the relative enrichment of the different constructs.

In order to compute IC50 curves and value, raw data were normalized using a min-max normalization (with positive and negative controls as max and min, Kevorkov and Makarenkov, 2005).

## RESULTS AND ANALYSIS:

While Nter and Cter fusion tags are useful tools for studying protein-RNA interactions, their specific localization and interaction with RNA could impact the results of these studies. We first examined the effect of the GFP fusion protein localization on the enrichment of mRNA on YB1 (Figure 10).

Our results showed that independently of the localization of the GFP tag (Nter or Cter) YB1 and RNA could still interact (Figure 11).

However, the enrichments are significantly different, with a lower enrichment for the Nter GFP tagged construct. The mechanisms of YB1:RNA interactions explaining this difference still need to be investigated.

Next, we examined the effect of deleting the cold-shock domain of YB1 (CSD)(SEQ ID N°23), a conserved RNA-binding domain that was found to be druggable (El Hage et al, under review). (Figure 12).

Our results showed that deletion of the RNA binding domain resulted in a significant decrease in RNA binding and enrichment for both the Nter and Cter GFP tagged proteins. These results indicate that the presence of the RNA binding domain is necessary for significant enrichment to occur.

Similarly to the PPI system, we tried to probe whether perturbations by small molecules could be quantified with the MT bench. Preliminary results in NMR and in cells showed that C8 compound decreases RNA:YB1 interaction. We determined the IC50 of this molecule in HeLa cells over 19 concentrations using the MT bench assay: 31.3 μM (Figure 13).

These combined results demonstrate that the MT bench technology is sensitive enough to detect variations in protein/RNA interactions. This technology combined to protein modifications/recombinations/mutations expressed in cells (via gateway vectors) will allow to characterize and quantify protein/RNA interactions in cellulo.

## Sequence Listing Free Text

| | | |
|---|---|---|
| SEQ ID NO 1 | Alpha tubulin (Accession: AAA91576) | MRECISIHVGQAGVQIGNACWELYCLEHGIQPDGQMPSDKTIGGGDDSFNTFF SETGAGKHVPRAVFVDLEPTVIDEVRTGTYRQLFHPEQLITGKEDAANYARGH YTIGKEIIDLVLDRIRKLADQCTRLQGFLVFHSFGGGTGSGFTSLLMERLSVDYG KSKLEFSIYPAPQVSTAVVEPYNSILTTHTTLEHSDCAFMVDNEAIYDICRRNLDI ERPTYTNLNRLISQIVSSITASLRFDGALNVDLTEFQTNLVPYPRIHFPLATYAPVI SAEKAYHEQLSVADITNACFEPANQMVKCDPGHGKYMACCLLYRGDVVPKDV NAAIATIKTKRTIQFVDWCPTGFKVGINYQPPTVVPGGDLAKVQRAVCMLSNTT AIAEAWARLDHKFDLMYAKRAFVHWYVGEGMEEGEFSEAREDMAALEKDYEE VGVDSVEGEGEEEGEEY |
| SEQ ID NO 2 | Beta tubulin (aa: 124-268, Accession: AAB59507): | MREIVHIQAGQCGNQIGAKFWEVISDEHGIDPTGTYHGDSDLQLDRISVYYNEA TGGKYVPRAILVDLEPGTMDSVRSGPFGQIFRPDNFVFGQSGAGNWAKGHYT EGAELVDSVLDVVRKEAESCDCLQGFQLTHSLGGGTGSGMGTLLISKIREEYP DRIMNTFSVVPSPKVSDTVVEPYNATLSVHQLVENTDETYCIDNEALYDICFRTL RLTTPTYGDLNHLVSGTMECVTTCLRFPGQLNADLRKLAVNMVPFPRLHFFMP GFAPLTSRGSQQYRALTVPDLTQQVFDAKMMAACDPRHGRYLTVAAVFRGR MSMKEVDEQMLNVQNKSSYFVEWIPNVKTAVCDIPPRGLKMAVTFIGNSTAIQ ELFKRISEQFTAMFRRKAFLHWYTGEGMDEMEFTEAESNMNDLVSEYQQYQD ATAEEEEDFGEEAEEEA |
| SEQ ID NO 3 | Tau projection domain (aa: 1-150, Accession: NP 005901.2): | MAEPRQEFEVMEDHAGTYGLGDRKDQGGYTMHQDQEGDTDAGLKESPLQT PTEDGSEEPGSETSDAKSTPTAEDVTAPLVDEGAPGKQAAAQPHTEIPEGTTA EEAGIG DTPSLEDEAAGHVTQARMVSKSKDGTGSDDKKAKGADGKTK |
| SEQ ID NO 4 | Tau microtubule binding sequence: 151-400, | IATPRGAAPPGQKGQANATRIPAKTPPAPKTPPSSGEPPKSGDRSGYSSPGSP GTPG SRSRTPSLPTPPTREPK VAVVRTPPKSPSSAKSRLQTAPVPMPDLK VKSKIGSTENLKHQPGGGGKVQIINKKLDLSNVQSKCGSKDNIKHVPGGGSVQIV YKPVDLSKVTSKCGSLGNIHHKPGGGQVEVKSEKLDFKDRVQSKIGSLDNITHV PGGGNKKIETHKLTFRENAKAKTDHGAEIVYKSPVVS |
| | Accession: NP 005901.2): | |
| SEQ ID NO 5 | Tau MBDIaa : 243-274: | LQTAPVPMPDLKNVKSKIGSTENLKHQPGGGK |
| SEQ ID NO 6 | Tau MBD2 aa : 275-305: | VQIINKKLDLSNVQSKCGSKDNIKHVPGGGS |
| SEQ ID NO 7 | Tau MBD3 aa : 306-336: | VQIVYKPVDLSKVTSKCGSLGNIHHKPGGGQ |
| SEQ ID NO 8 | Tau MBD4 aa : 337-368 | VEVKSEKLDFKDRVQSKIGSLDNITHVPGGGN |
| SEQ ID NO 9 | MAPI A (aa: 282-630. Accession: NP 002364) | QNKILEGLEKLRHLDFLRYPVATQKDLASGAVPTNLKPSKIKQRADSKESLKAT TKTAVSKLAKREEVVEEGAKEARSELAKELAKTEKKAKESSEKPPEKPAKPER VKTESSEALKAEKRKLIKDKVGKKHLKEKISKLEEKKDKEKKEIKERKELKKDEG RKEEKKDAKKEEKRKDTKPELKKISKPDLKPFTPEVRKTLYKAKVPGRVKIDRS RAIRGEKELSSEPQTPPAQKGTVPLPTISGHRELVLSSPEDLTQDFEEMKREER ALLAEQRDTGLGDKPFPLDTAEEGPPSTAIQGTPPSVPGLGQEEHVMKEKELV PEVPEEQGSKDRGLDSGAETEEEKDTWEEKKQRE |
| SEQ ID NO 10 | MAP2 (aa: 1519-1828, Accession: NP 002365) | FKQAKDKVSDGVTKSPEKRSSLPRPSSILPPRRGVSGDRDENSFSLNSSISSS ARRTTRSEPIRRAGKSGTSTPTTPGSTAITPGTPPSYSSRTPGTPGTPSYPRTP HTPGTPKSAILVPSEKVAIIRTPPKSPATPKQLRLINQPLPDLKVKSKIGSTDNIKY QPKGGGQVQIVTKKIDLSHVTSKCGSLKIRHRPGGGRVKIESVKLDFKEKAQAKV GSLDNAHHVPGGGNVKIDSQKLNFREHAKARVDHGAEIITQSPGRSSVASPRR LSNVSSSGSINLLESPQLATLAEDVTAALAKQGL |

(continued)

| SEQ ID NO 11 | MAP4 (aa: 923-1084. Accession: AAA67361) | LATNTSAPDLKVRSKVGSTENIKHQPGGGRAKVEKTEAAATTRKPESNAVTKT AGPIASAQKQPAGKVQIVSKVSYSHIQSKCGSKDNIKHVPGGGNVQIQNKVDIS KVSSKCGSKANIKHKPGGGDVKIESQKLNFKEKAQAKVGSLDNVGHLPAGG |
|---|---|---|
| SEQ ID NO 12 | MAP6 (aa: 118-321, Accession: NP 149052) | SVMRQDYRAWKVQRPEPSCRPRSEYQPSDAPFERETQYQKDFRAWPLPRR GDHPWIPKPVQISAASQASAPILGAPKRRPQSQERWPVQAAAEAREQEAAPG GAGGLAAGKASGADERDTRRKAGPAWIVRRAEGLGHEQTPLPAAQAQVQAT GPEAGRGRAAADALNRQIR EEVASAVSSSYRNEFRAWTDIKPVKPIKAKP |
| SEQ ID NO 13 | EB1 (aa: 124-268, Accession: NP 036457) | YDPVAARQGQETAVAPSLVAPALNKPKKPLTSSSAAPQRPISTQRTAAAPKAG PGVVRKPGVGNGDDEAAELMQQVNVLKLTVEDLEKERDFYFGKLRNIELICQE NEGENDPVLQRIVDILYATDEGFVIPDEGGPQEEQEEY |
| SEQ ID NO 14 | MBD/ « tau » | MAEPRQEFEVMEDHAGTYGLGDRKDQGGYTMHQDQEGDTDAGLKESPLQT PTEDGSEEPGSETSDAKSTPTAEDVTAPLVDEGAPGKQAAAQPHTEIPEGTTA EEAGIGDTPSLEDEAAGHVTQARMVSKSKDGTGSDDKKAKGADGKTKIATPR GAAPPGQKGQANATRIPAKTPPAPKTPPSSGEPPKSGDRSGYSSPGSPGTPG SRSRTPSLPTPPTREPKKVAVVRTPPKSPSSAKSRLQTAPVPMPDLKNVKSKI GSTENLKHQPGGGKVQIINKKLDLSNVQSKCGSKDNIKHVPGGGSVQIVYKPV DLSKVTSKCGSLGNIHHKPGGGQVEVKSEKLDFKDRVQSKIGSLDNITHVPGG GNKKIETHKLTFRENAKAKTDHGAEIVYKSPVVSGDTSPRHLSNVSSTGSIDMV DSPQLATLADEVSASLAKQGL |
| SEQ ID NO 15 | P53-FL | MEEPQSDPSVEPPLSQETFSDLWKLLPENNVLSPLPSQAMDDLMLSPDDIEQ WFTEDPGPDEAPRMPEAAPVAPAPAAPTPAAPAPAPSWPLSSSVPSQKTYQ GSYGFRLGFLHSGTAKSVTCTYSPALNKMFCQLAKTCPVQLWVDSTPPPGTR VRAMAIYKQSQHMTEVVRRCPHHERCSDSDGLAPPQHLIRVEGNLRVEYLDD RNTFRHSVVVPYEPPEVGSDCTTIHYNYMCNSSCMGGMNRRPILTIITLEDSSG NLLGRNSFEVRVCACPGRDRRTEEENLRKKGEPHHELPPGSTKRALPNNTSS SPQPKKKPLDGEYFTLQIRGRERFEMFRELNEALELKDAQAGKEPGGSRAHSS HLKSKKGQSTSRHKKLMFKTEGPDSD |
| SEQ ID NO 16 | p53W23A | MEEPQSDPSVEPPLSQETFSDLAKLLPENNVLSPLPSQAMDDLMLSPDDIEQW FTEDPGPDEAPRMPEAAPVAPAPAAPTPAAPAPAPSWPLSSSVPSQKTYQG SYGFRLGFLHSGTAKSVTCTYSPALNKMFCQLAKTCPVQLWVDSTPPPGTRV RAMAIYKQSQHMTEVVRRCPHHERCSDSDGLAPPQHLIRVEGNLRVEYLDDR NTFRHSVVVPYEPPEVGSDCTTIHYNYMCNSSCMGGMNRRPILTIITLEDSSGN LLGRNSFEVRVCACPGRDRRTEEENLRKKGEPHHELPPGSTKRALPNNTSSS PQPKKKPLDGEYFTLQIRGRERFEMFRELNEALELKDAQAGKEPGGSRAHSS HLKSKKGQSTSRHKKLMFKTEGPDSD |
| SEQ ID NO 17 | p53F19A | MEEPQSDPSVEPPLSQETASDLWKLLPENNVLSPLPSQAMDDLMLSPDDIEQ WFTEDPGPDEAPRMPEAAPVAPAPAAPTPAAPAPAPSWPLSSSVPSQKTYQ GSYGFRLGFLHSGTAKSVTCTYSPALNKMFCQLAKTCPVQLWVDSTPPPGTR VRAMAIYKQSQHMTEVVRRCPHHERCSDSDGLAPPQHLIRVEGNLRVEYLDD RNTFRHSVVVPYEPPEVGSDCTTIHYNYMCNSSCMGGMNRRPILTIITLEDSSG NLLGRNSFEVRVCACPGRDRRTEEENLRKKGEPHHELPPGSTKRALPNNTSS |
| | | SPQPKKKPLDGEYFTLQIRGRERFEMFRELNEALELKDAQAGKEPGGSRAHSS HLKSKKGQSTSRHKKLMFKTEGPDSD |
| SEQ ID NO 18 | p53F19AW23 A | MEEPQSDPSVEPPLSQETASDLAKLLPENNVLSPLPSQAMDDLMLSPDDIEQW FTEDPGPDEAPRMPEAAPVAPAPAAPTPAAPAPAPSWPLSSSVPSQKTYQG SYGFRLGFLHSGTAKSVTCTYSPALNKMFCQLAKTCPVQLWVDSTPPPGTRV RAMAIYKQSQHMTEVVRRCPHHERCSDSDGLAPPQHLIRVEGNLRVEYLDDR NTFRHSVVVPYEPPEVGSDCTTIHYNYMCNSSCMGGMNRRPILTIITLEDSSGN LLGRNSFEVRVCACPGRDRRTEEENLRKKGEPHHELPPGSTKRALPNNTSSS PQPKKKPLDGEYFTLQIRGRERFEMFRELNEALELKDAQAGKEPGGSRAHSS HLKSKKGQSTSRHKKLMFKTEGPDSD |

(continued)

| SEQ ID NO 19 | p53W23AL26 A | MEEPQSDPSVEPPLSQETFSDLAKLLPENNVLSPLPSQAMDDLMLSPDDIEQW FTEDPGPDEAPRMPEAAPPVAPAPAAPTPAAPAPAPSWPLSSSVPSQKTYQG SYGFRLGFLHSGTAKSVTCTYSPALNKMFCQLAKTCPVQLWVDSTPPPGTRV RAMAIYKQSQHMTEVVRRCPHHERCSDSDGLAPPQHLIRVEGNLRVEYLDDR NTFRHSVVVPYEPPEVGSDCTTIHYNYMCNSSCMGGMNRRPILTIITLEDSSGN LLGRNSFEVRVCACPGRDRRTEEEENLRKKGEPHHELPPGSTKRALPNNTSSS PQPKKKPLDGEYFTLQIRGRERFEMFRELNEALELKDAQAGKEPGGSRAHSS HLKSKKGQSTSRHKKLMFKTEGPDSD |
| SEQ ID NO 20 | p53F19AW23 AL26A | MEEPQSDPSVEPPLSQETFSDLAKLAPENNVLSPLPSQAMDDLMLSPDDIEQW FTEDPGPDEAPRMPEAAPPVAPAPAAPTPAAPAPAPSWPLSSSVPSQKTYQG SYGFRLGFLHSGTAKSVTCTYSPALNKMFCQLAKTCPVQLWVDSTPPPGTRV RAMAIYKQSQHMTEVVRRCPHHERCSDSDGLAPPQHLIRVEGNLRVEYLDDR NTFRHSVVVPYEPPEVGSDCTTIHYNYMCNSSCMGGMNRRPILTIITLEDSSGN LLGRNSFEVRVCACPGRDRRTEEEENLRKKGEPHHELPPGSTKRALPNNTSSS PQPKKKPLDGEYFTLQIRGRERFEMFRELNEALELKDAQAGKEPGGSRAHSS HLKSKKGQSTSRHKKLMFKTEGPDSD |
| SEQ ID NO 21 | MDM2-FL | MCNTNMSVPTDGAVTTSQIPASEQETLVRPKPLLLKLLKSVGAQKDTYTMKEV LFYLGQYIMTKRLYDEKQQHIVYCSNDLLGDLFGVPSFSVKEHRKIYTMIYRNLV VVNQQESSDSGTSVSENRCHLEGGSDQKDLVQELQEEKPSSSHLVSRPSTSS RRRAISETEENSDELSGERQRKRHKSDSISLSFDESLALCVIREICCERSSSSES TGTPSNPDLDAGVSEHSGDWLDQDSVSDQFSVEFEVESLDSEDYSLSEEGQE LSDEDDEVYQVTVYQAGESDTDSFEEDPEISLADYWKCTSCNEMNPPLPSHC NRCWALRENWLPEDKGKDKGEISEKAKLENSTQAEEGFDVPDCKKTIVNDSR ESCVEENDDKITQASQSQESEDYSQPSTSSSIIYSSQEDVKEFEREETQDKEES VESSLPLNAIEPCVICQGRPKNGCIVHGKTGHLMACFTCAKKLKKRNKPCPVC RQPIQMIVLTYFP |
| SEQ ID NO 22 | YB1-FL | MSSEAETQQPPAAPPAAPALSAADTKPGTTGSGAGSGGPGGLTSAAPAGGD KKVIATKVLGTVKWFNVRNGYGFINRNDTKEDVFVHQTAIKKNNPRKYLRSVG DGETVEFDVVEGEKGAEAANVTGPGGVPVQGSKYAADRNHYRRYPRRRGPP RNYQQNYQNSESGEKNEGSESAPEGQAQQRRPYRRRRFPPYYMRRPYGRR PQYSNPPVQGEVMEGADNQGAGEQGRPVRQNMYRGYRPRFRRGPPRQRQ PREDGNEEDKENQGDETQGGQQPPQRRYRRNFNYRRRRPENPKPQDGKETK AADPPAENSSAPEAEQGGAE |
| SEQ ID NO 23 | YB1-deltaCSD | MSSEAETQQPPAAPPAAPALSAADTKPGTTGSGAGSGGPGGLTSAAPAGGD GVPVQGSKYAADRNHYRRYPRRRGPPRNYQQNYQNSESGEKNEGSESAPE GQAQQRRPYRRRRFPPYYMRRPYGRRPQYSNPPVQGEVMEGADNQGAGE QGRPVRQNMYRGYRPRFRRGPPRQRQPREDGNEEDKENQGDETQGGQQPP QRRYRRNFNYRRRRPENPKPQDGKETKAADPPAENSSAPEAEQGGAE |

## Claims

1. An *in vitro* method for evaluating the ability of a compound to disrupt or stabilize an interaction between one or more bait and one or more candidate prey in a eukaryotic cell, comprising the steps of:

   a. providing a eukaryotic cell expressing (i) one or more bait, and (ii) one or more candidate prey, wherein said bait comprises a bait moiety and a polymerized tubulin-binding moiety;
   b. determining the occurrence of an interaction between said one or more bait and said one or more candidate prey in the eukaryotic cell, wherein said bait is bound to polymerized tubulin in the eukaryotic cell, thereby localizing said one or more candidate prey along said polymerized tubulin, thereby detecting said interaction;
   c. contacting the said eukaryotic cell with the said compound;
   d. determining the occurrence of the interaction between said one or more bait and said one or more candidate prey in the eukaryotic cell as in step b. and quantify this interaction;
   e. Comparison of the occurrences of the interaction made in step b. and in. step d.;
   f. Conclude whether the effect of the said compound is the disruption or the stabilization of the interaction between said one or more bait and one or more candidate prey.

2. The *in vitro* method according to claim 1, wherein said bait comprises:

   - a polymerized tubulin-binding moiety comprising one or more Microtubule-Binding Domain (MBD), and
   - a bait moiety.

3. The *in vitro* method according to any one of claims 1 or 2, wherein said bait comprises one Linker (L) region located between the polymerized-tubulin binding moiety and the bait moiety.

4. The *in vitro* method according to any one of the preceding claims, wherein the candidate prey comprises a fluorescent protein.

5. The *in vitro* method according to any one of the preceding claims, wherein in step b. and d. determining the occurrence of said prey in the eukaryotic cell, is a detection method selected in a group comprising: binding to an antibody, hybridization with a nucleic acid, fluorescence measurement.

6. The *in vitro* method according to any one of the preceding claims, wherein the cell in step b. is a fixed cell or a living cell.

7. The *in vitro* method according to any one of the preceding claims, wherein the method is repeated with different concentrations of the compound in order to determine the IC50 of the compound for the tested combination of one or more bait and one or more candidate prey.

8. The *in vitro* method according to any one of the preceding claims, wherein steps a. and b. are replicate before step c. with different vector constructs of said one or more bait and said one or more candidate prey, in order to determine the occurrence of the interactions between said one or more bait and said one or more candidate prey in the eukaryotic cell, and choose the right combination to be evaluate in the following steps to be closer to what happens in vivo.

9. The *in vitro* method according to any one of the preceding claims, wherein the said one or more candidate prey and/or said one or more bait are chosen among: ribonucleic acid and/or deoxyribonucleic acid and/or protein.

10. The *in vitro* method according to any one of claims 1 to 9, wherein the said one or more bait is a protein.

11. The *in vitro* method according to any one of claims 1 to 10, wherein the said one or more candidate prey is a ribonucleic acid.

12. The *in vitro* method according to any one of claims 1 to 10, wherein the said one or more candidate prey is a protein.

13. The *in vitro* method according to any one of preceding claims, wherein the one or more bait and one or more candidate prey are characteristics of a proteinopathy or of a cancer.

14. A compound identified by the *in vitro* method according to any one of the preceding claims, for use as a medicament.

15. Use of a polymerized-tubulin binding moiety fused to one or more bait, as a tool for evaluating the ability of a compound to disrupt or stabilize an interaction between said one or more bait and one or more prey in a eukaryotic cell, wherein said bait is bound to polymerized tubulin in the eukaryotic cell, thereby localizing said one or more candidate prey along said polymerized tubulin, the said one or more bait and the said one or more candidate prey being contacted with the said compound, thereby allowing the evaluation of the disruption or stabilization of said interaction.

[Fig. 1]

Transfection of cell samples

Basic fluorescence imaging

YB-1-GFP-tau    Cy3-labelled poly-T probes

Image of the bait
YB-1-GFP-tau is bound to
microtubules

Image of the prey:
mRNA using Poly-T
with Cy3 probe

Microtubular structures
in the prey image are
clearly observed in the
cell expressing YB-1-
GFP-tau

YB-1-GFP

Control: YB-1-GFP
is homogeneously
distributed

Detection

[Fig. 2]

[Fig. 3]

Step 1: Co-transfection

Plasmid 1 :
Expression of bait
in eukaryotic cells

Plasmid 2 :
Expression of prey in
eukaryotic cells (the label
can be placed either at
the C-terminal or
N-terminal end)

24 to 72 h

Step 2 : Imaging of prey (GFP
or antibody) in the cytoplasm

Prey

Bait

Prey

Microtubule binding
domain of MAPs

Protein (used as bait)

Unstructured projection
domain of MAPs

Bait

Dynamical
Microtubule

Dynamical
Microtubule

**No interaction between
bait and prey**

**Interaction between
bait and prey**

Detection of prey brought to microtubules
(Fluorescent label or antibody)

[Fig. 4]

[Fig. 5]

[Fig. 6]

[Fig. 7]

[Fig. 8]

| Figure legend | Bait | Prey | Inhibitor |
|---|---|---|---|
| Ctrl neg (FL) | P53-FL_C_RFP-MBD[1.2ug] | MDM2-FL_C_GFP[0.2ug] | RG-7112[5uM] |
| F19A | P53-F19A_C-RFP-MBD[1.2ug] | MDM2-FL_C_GFP[0.2ug] | No |
| W23A | P53-W23A_C_RFP-MBD[1.2ug] | MDM2-FL_C_GFP[0.2ug] | No |
| L26A | P53-L26A-C-RFP-MBD[1.2ug] | MDM2-FL_C_GFP[0.2ug] | No |
| F19AW23A | P53-F19AW23A_C_RFP-MBD[1.2ug] | MDM2-FL_C_GFP[0.2ug] | No |
| W23AL26A | P53-W23AL26A_C_RFP-MBD[1.2ug] | MDM2-FL_C_GFP[0.2ug] | No |
| F19AW23AL26A | P53-F19AW23AL26A_C_RFP-MBD[1.2ug] | MDM2-FL_C_GFP[0.2ug] | No |
| Ctrl pos (FL) | P53-FL_C_RFP-MBD[1.2ug] | MDM2-FL_C_GFP[0.3ug] | No |

[Fig. 9]

[Fig. 10]

MBD-GFP-YB1-FL   YB1-FL-GFP-MBD

[Fig. 11]

[Fig. 12]

[Fig. 13]

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EL HAGE KRYSTEL ET AL: "Targeting RNA:protein interactions with an integrative approach leads to the identification of potent YBX1 inhibitors", UNIVERSITÉ PARIS-SACLAY, INSERM U1204, UNIV EVRY, STRUCTURE-ACTIVITÉ DES BIOMOLÉCULES NORMALES ET PATHOLOGIQUES (SABNP), vol. 12, 17 January 2023 (2023-01-17), XP093086095, DOI: 10.7554/eLife.80387 Retrieved from the Internet: URL:https://cdn.elifesciences.org/articles/80387/elife-80387-v2.xml> * p. 6, section "Robust HCS scoring of endogenous mRPIs in cells with the MT bench assay " to p. 9, section "Identification of potent mRNA:YB-1 interaction inhibitors in cells".; figure 4 * | 1-13,15 | INV. G01N33/58 G01N33/68 |
| X | BOCA MIRELA ET AL: "Probing protein interactions in living mammalian cells on a microtubule bench", SCIENTIFIC REPORTS, vol. 5, no. 1, 27 November 2015 (2015-11-27), XP93086047, DOI: 10.1038/srep17304 Retrieved from the Internet: URL:https://www.nature.com/articles/srep17304.pdf> * the whole document * | 1-13,15 | TECHNICAL FIELDS SEARCHED (IPC) G01N |
| X,D | WO 2016/012451 A1 (INST NAT SANTE RECH MED [FR]; UNIV D EVRY VAL D ESSONNE [FR]) 28 January 2016 (2016-01-28) * the whole document * | 1-13,15 | |

-/--

The present search report has been drawn up for all claims

| Place of search | Date of completion of the search | Examiner |
|---|---|---|
| Munich | 26 September 2023 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches
Patentamt

European
Patent Office

Office européen
des brevets

# EUROPEAN SEARCH REPORT

Application Number

EP 23 17 1184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/229068 A1 (YAO ZHONG [CA] ET AL) 21 July 2022 (2022-07-21) * claim 20 * ----- | 1-13,15 | |
| A | WO 2021/203016 A2 (UNIV CALIFORNIA [US]; EINDHOVEN UNIV OF TECHNOLOGY [NL] ET AL.) 7 October 2021 (2021-10-07) * the whole document * ----- | 1-13,15 | |

TECHNICAL FIELDS
SEARCHED      (IPC)

The present search report has been drawn up for all claims

| Place of search | Date of completion of the search | Examiner |
|---|---|---|
| Munich | 26 September 2023 | Jacques, Patrice |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 17 1184

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2023

| Patent document cited in search report | | Publication date | Patent family member(s) | | Publication date |
|---|---|---|---|---|---|
| WO 2016012451 | A1 | 28-01-2016 | EP | 3172563 A1 | 31-05-2017 |
| | | | ES | 2739525 T3 | 31-01-2020 |
| | | | US | 2017242026 A1 | 24-08-2017 |
| | | | WO | 2016012451 A1 | 28-01-2016 |
| US 2022229068 | A1 | 21-07-2022 | CA | 3109167 A1 | 07-01-2021 |
| | | | EP | 3994251 A1 | 11-05-2022 |
| | | | US | 2022229068 A1 | 21-07-2022 |
| | | | WO | 2021000043 A1 | 07-01-2021 |
| WO 2021203016 | A2 | 07-10-2021 | EP | 4125861 A2 | 08-02-2023 |
| | | | US | 2023142739 A1 | 11-05-2023 |
| | | | WO | 2021203016 A2 | 07-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016012451 A

### Non-patent literature cited in the description

- **SALMON ; WATERMAN.** How we discovered fluorescent speckle microscopy. *Mol Biol Cell,* 2011, 3940-2
- **CRAVCHIK et al.** Identification of a novel microtubule-binding domain in microtubule-associated protein 1A (MAP1A). *J Cell Sci,* 1994, vol. 107, 661-72
- **SHANER ; STEINBACH ; TSIEN.** A guide to choosing fluorescent proteins. *Nat Methods,* 2005, vol. 2 (12), 905-9
- **ALTINOK et al.** Activity analysis in microtubule videos by mixture of hidden Markov models; Computer Vision and Pattern Recognition. *IEEE Computer Society Conference,* 2006, vol. 2, 1662-1669
- **OCHOA et al.** Cold exposure reveals two populations of microtubules in pulmonary endothelia. *Am. J. physiol. Lung Cell. Mol. Physiol,* 2011, vol. 300, L132-L138
- **DESFORGES et al.** An intercellular polyamine transfer via gap junctions regulates proliferation and response to stress in epithelial cells. *Mol Biol Cell,* 2013, vol. 24, 1529-1543